# EUROPEAN PATENT APPLICATION

(11) **EP 4 342 435 A1**
(43) Date of publication of application: **27.03.2024**
(21) Application number: 23172062.4
(22) Date of filing: 08.05.2023
(51) Int. Cl.: A61F 9/008, A61F 9/007

(54) **LASER SYSTEM AND METHOD FOR DETECTING AND PROCESSING INFORMATION**

(30) Priority: 23.09.2022 EP 22197529; 18.11.2022 EP 22208402
(71) Applicant: Terra Quantum AG, 9000 St. Gallen (CH)
(72) Inventor: Sobol, Emil, 9000 St. Gallen (CH); Vinokour, Valerii, 9000 St. Gallen (CH)
(74) Representative: Kretschmann, Dennis

(57) **Abstract**

The present disclosure provides a laser system suitable for changing an IOP of an eye, the laser system comprising: a laser source; a feedback controller, configured to regulate a dosimetry of the laser source to produce spatially and/or temporally modulated laser light; a first optical delivery element, configured to guide the spatially and/or temporally modulated laser light to irradiate a first area on the eye; and a detecting element, configured to detect one or more physical, chemical, mechanical and/or structural characteristics in a second area on the eye in a real-time during the change of the IOP, wherein the feedback controller is configured to regulate the dosimetry of the laser source in a real-time based on the real-time detected information pertaining to the one or more physical, chemical, mechanical and/or structural characteristics in the second area.

## Description

### Field of the Disclosure

The present disclosure is in the field of a medical laser technology. In particular, the disclosure relates to a laser system that may be applied for the normalization of intraocular pressure (IOP) in eyes with glaucoma.

### Background

Glaucoma is characterized by optic nerve damage that frequently leads to irreversible vision loss. Glaucoma is a leading cause of irreversible blindness worldwide. There are over 80 million people worldwide with glaucoma, and this number is expected to increase to 110 million by 2040. It has been estimated that Glaucoma costs the U.S. economy $2.86 billion every year in direct costs and productivity losses.

An important element in glaucoma pathophysiology is the secretion, transport, and eventual outflow of aqueous humor from the anterior chamber of the eye. Raised intraocular pressure (IOP) is a significant risk factor for developing glaucoma. IOP is kept in balance through the production of aqueous humor by the ciliary body of the eye and its drainage through several outflow pathways including the conventional pathway, namely through trabecular meshwork (TM) and Schlemm's canal; and the uveoscleral pathway, namely through the sclera.

Currently, glaucoma therapies aim at reducing IOP by either limiting the production of aqueous humor or by increasing the outflow of aqueous humor. Treatments for glaucoma include medications, usually eye drops, to help eye fluid drain more effectively or lessen fluid production; laser surgery; and conventional surgery.

Trabeculectomy has been the standard surgical procedure for 40 years but carries known risks of cataract, hypotony, bleb failure, infection, and other complications. Various value procedures have been combined with trabeculectomy to shunt aqueous into the sub-tenon's space (e.g., Ahmed valve). Recently, microinvasive glaucoma surgery has been introduced such as the iStent (Glaukos, Laguna Hills, CA) and Trabectome (NeoMedix, Tustin, CA). The CyPass (Transcend Medical, Menlo Park, CA) works on the suprachoroidal space and the uveoscleral area. It allows lowering of IOP without utilization of the conventional transport pathway. All above approaches to control IOP in glaucomatous eyes have problematic complications.

Laser trabeculoplasty (LTP) and selective laser trabeculoplasty (SLT) are in common practice now, but it can lead to irreversible changes in Schlemm's canal and other secondary effects. SLT is a subclass of laser treatments for IOP reduction that targets the TM and improves aqueous outflow.

Since the mechanism of action of SLT is not fully understood, it is difficult to predict success rate or prevent complications. SLT has a variable success rate of around 40%-70%.

Although SLT is relatively safe and efficacious, the possible complications include an elevated IOP at one to two hours after treatment (the duration of IOP elevation may last between 4 days and 3 months), corneal haze, and shifts in refractive error (both myopic and hyperopic). Postoperative inflammation following SLT usually occurs 2 to 3 days following the procedure. It has been seen in 83% of eyes undergoing SLT. There have been reports of TM cell death and retinal side effects.

Another common operation is laser cyclocoagulation (LCC), based on local coagulation (destruction) of the ciliary body, which reduces aqueous humor production and decreases IOP. The outcome of LCC is often difficult to accurately predict. Moreover, this method can have serious complications, including in particular an excessive decrease in IOP and/or some increase in the central thickness of the cornea, which may lead to decompensation of the corneal endothelium. LCC is currently reserved mostly for cases of patients with highly advanced glaucoma and poor vision.

Therefore, the existing treatment modalities for glaucoma, including pharmacological therapy, lasers, surgery, and shunts, all have certain shortcomings. In addition, all available methods and devices are developed and used for IOP reduction. None of the above-mentioned methods can treat low IOP glaucoma.

The prior art and knowledge are summarized in the following patents and publications:
- Baum, O., et al. "Laser-induced hypotensive effect in treatment of the resistant open-angle glaucoma." Optical Interactions with Tissue and Cells XXXII. Vol. 11640. SPIE, 2021. discloses the scientific background of the present disclosure. It illustrates the mechanism that enables forming a stabilized microporous structure on sclera in vivo using temporally and spatially modulated laser.
- US 2022/0175580 A1 discloses a controlled laser for prophylactic treatment for preventing bleeding from subsequently formed incisions.
- US 11,058,890 B2 discloses a method for adjusting a laser system in a cyclophotocoagulation treatment.
- Goldenfeld, Mordechai, et al. "Automated direct selective laser trabeculoplasty: first prospective clinical trial." Translational Vision Science & Technology 10.3 (2021): 5-5. discloses an DSLT system automated eye-tracking system.
- Further technical backgrounds for the present disclosure can be found in the review paper: Song, Julia. "Complications of selective laser trabeculoplasty: a review." Clinical Ophthalmology (2016): 137-143. and the review paper: Radcliffe, Nathan, et al. "Energy Dose-Response in Selective Laser Trabeculoplasty: A Review." Journal of Glaucoma 31.8 (2022): e49.

### Summary of the Disclosure

The objective of the present disclosure is to provide a device and a method that solve one or more of the above-mentioned problems of the prior art. The present disclosure is defined by the appended claims.

A first aspect of the disclosure provides a laser system suitable for changing an intraocular pressure (IOP) of an eye, the laser system comprising: a laser source; a feedback controller, configured to regulate a dosimetry of the laser source to produce spatially and/or temporally modulated laser light; a first optical delivery element, configured to guide the spatially and/or temporally modulated laser light to irradiate a first area on the eye; and a detecting element, configured to detect one or more physical, chemical, mechanical and/or structural characteristics in a second area on the eye in a real-time during the change of the IOP, wherein the feedback controller is configured to regulate the dosimetry of the laser source in a real-time based on the real-time detected information pertaining to the one or more physical, chemical, mechanical and/or structural characteristics in the second area.

In an operation scenario of the laser system, a medical doctor or a medical practitioner may introduce the first optical delivery element to the vicinity of the first area manually. A doctor or practitioner may then select a function of the laser system, for example to determine the first area or to determine whether the IOP needs to be increased or decreased. The doctor or practitioner may then set one of the preset laser modes (dosimetry parameters) and start the laser treatment. After the laser treatment is started, it can run automatically until a detected information reaches a predetermined threshold, for example, when the IOP reaches a predetermined value. When such a threshold is reached, the controller can either stop the laser treatment or pause and wait for the next command of a doctor or practitioner.

In the context of the present disclosure, "a real-time" may generally refer to a time scale in which the one or more physical, chemical, mechanical and/or structural characteristics on the eye are detected and are subsequently processed, wherein the time scale is sufficiently short to allow the dosimetry of the laser source to be purposefully regulated via the feedback based on the detected and processed information during the ongoing treatment of the eye, in particular, during the change of IOP.

In the context of the present disclosure, "a real-time" may refer to a time scale smaller than several minutes. For example, detecting in a real-time may refer to detecting continuously over a time period of several minutes or detecting several minutes after an external effect (such as a laser effect) for evaluation of such an effect. Processing in a real-time may refer to a processing where the result can be calculated several minutes after the calculation starts. Nonetheless, a smaller time scale is likewise possible.

In particular, a real-time may refer to a time scale smaller than 20 minutes, in particular smaller than 10 minutes or smaller than 3 minutes or smaller than 1 minute or smaller than 30 seconds or smaller than 10 seconds or smaller than 1 second.

In the context of the present disclosure, "regulating a dosimetry of a laser" or "regulating a laser" may refer to regulating the laser in operation. However, they may also comprise selecting a proper initial parameter of the laser for starting the laser treatment. In that case, detecting in a real-time may refer to the situation where the characteristics are detected within a time span of up to several minutes before the laser starts to work. The laser may start in different initial conditions depending on the exact situation of the to-be-treated eye.

In an implementation of the laser system of the first aspect, the laser may be regulated by adjusting at least one of the following laser parameters: a laser pulse repetition rate;; a duration of a laser pulse; a shape of a laser signal in the time domain; a shape of the laser signal in the frequency domain; a laser wavelength; a pulse energy; an intensity of the laser signal; a number of pulses in a pulse series; an interval duration between series; a number of total series; a spatial distribution of laser irradiation intensity; a dimension of irradiated area; a distance between neighboring irradiated areas; and a distance shift due to the propagation in the first optical delivery element.

Adjusting one or more of these parameters may facilitate a fine tuning based on the environment properties, which may increase the precision of the laser light modulation and the range of applications.

A real-time regulation of the dosimetry of the laser source may correspond to a constant adjustment of a laser dosimetry, adjusting the laser dosimetry upon receiving a signal from the feedback controller or updating the laser dosimetry after a certain number of pulses in a sequence. A real-time regulating may further comprise stopping the irradiation when the real-time detected information pertaining to the characteristic in the area reaches a predetermined or calculated threshold.

In a further implementation of the laser system of the first aspect, the detecting element may comprise at least one of the following: a pneumatic device; an IR radiometry; a photoacoustic detector; an OCE device; an OCT device; and a device for detecting backlight scattering.

These types of detecting elements may provide high resolution monitoring of the area or a local environment but may generate a large volume of data. Through combining a plurality of different types of the detecting elements with a powerful (built-in or external) computer, for example a quantum computer, the disclosure may facilitate a precise real-time control of the laser regulation.

In a further implementation of the laser system of the first aspect, the characteristics may comprise one or more of the following: IOP of the eye; a temperature; a temperature distribution; a pressure; a pressure distribution; a Young's modulus; a speed of sound; a chemical composition; a thickness, for example a thickness of sclera; a dimension, for example a dimension of ciliary body, Schlemm's canal and/or TM; a dimension of an unblocked lumen area, for example, a dimension of an unblocked lumen area in a humor drain pathway; a pore size; a pore size distribution.

In an example, due to the complexity of laser system, as well as the complicated environment in the human body, the desired result may be achieved through regulating multiple dosimetry parameters of the laser source at the same time based on a calculation of the real-time detected information pertaining to multiple characteristics in the area through a specially designed algorithm.

Due to the large number of input parameters and output parameters of the algorithm, a large feedback lag may lead to a divergence from the desired effect. Real-time control may be achieved by high-performance computing power. In an example, such a computer can be a (remote) high-performance computer, a (remote) hybrid quantum-classical computational facility, and/or a (remote) quantum computer.

Based on the real-time detected information pertaining to the one or more physical, chemical, mechanical and/or structural characteristics on the eye, a real-time spatially and/or temporally modulated laser light targeting the eye may facilitate a controllable noninvasive treatment to modulate the IOP.

In an implementation of the laser system of the first aspect, the first area may comprise a part on a sclera of the eye, and wherein the laser source may be regulated to modify a porous structure on the sclera.

Conventional laser systems for changing IOP of eyes focus on the conventional pathway or coagulating ciliary body. The importance of the uveoscleral pathway is usually overlooked. By modifying porous structures on the sclera, the present disclosure provides a novel solution for adjusting the aqueous humor outflow. This may be especially useful if the option to treat the other parts of the eye is no longer available, for example, when the Schlemm's canal and TM have been treated before and can no longer be treated again. In addition, forming and adjusting a porous structure on the sclera largely preserves the integrity and mechanical stability of the eye, leading to little negative effect.

In the context of the present disclosure, a porous structure may refer to a structure with a distributed plurality of structural defects. A pore in such a porous structure does not need to be of a rounded shape in a conventional sense, but can also be a creek, microcavity, displacement, or another form of a structural defect that promote transfer of aqueous humor through the tissue matrix. Yet a porous structure should also be distinguished from an assembly of macrofractures, that impairs the mechanical properties of the tissue. In an exemplary configuration, the porous structure may be a microporous structure, that is to say, the structural defects can have the size less than 5 micrometers. At the initial stage of formation of such a microporous structure, the formation may only modify the physical and chemical properties of the affected area and may not significantly change the macroscopic appearance and mechanical properties of the tissues.

In some examples, the tissue and/or the object on which the porous structure is formed may already be porous before the laser-induced porous formation. In these examples, a porous structure refers to a structure with an increased porosity over the untreated tissue or object. In some examples, a porous structure formation may refer to an increase in the porosity and/or an increase in the pore sizes. In another example, a porous structure formation may refer to a de-clogging of a preexisting clogged porous structure. Since clogging may often occur in an unstable porous structure, the controlled porous structure formation according to the present disclosure may be applied to de-clog the clogged unstable porous structure and form a stabilized porous structure for a long-time stability.

In an example, the size of the pores, or the porosity of the porous structure, can be controlled according to the desired aqueous humor outflow. In another example, the size of the pores can be small enough to maintain a structural integrity of the sclera and/ or to maximize the stability of the pores.

In a further implementation of the laser system of the first aspect, the detecting element may comprise an optical receiving element.

In a further implementation of the laser system of the first aspect, the optical receiving element may be configured to receive a scattered light.

The scattered light may stem from the spatially and/or temporally modulated laser light. Light scattering may be sensitive to the formation of microporous structures, or other defects on a microscopic level. Detecting and analyzing the scattered light of different wavelengths makes it possible to use the Mie and Rayleigh scattering laws, to determine the size distributions of pores, defects, or potential gas bubbles on the sclera, which may be generated during the porous structure formation.

In a further implementation of the laser system of the first aspect, the detecting element may comprise a second optical delivery element.

In a further implementation of the laser system of the first aspect, the second optical delivery element may be configured to deliver a probing light signal for light scattering analysis.

Scattered light may also stem from a probing light signal. The probing light signal needs not to interact with the tissue or the object so as to form pores and can be used before the laser operation to determine the initial condition of the laser. This may facilitate an initial condition of the laser with little or no destructive side effect on the tissue or the object or their environment.

In a further implementation of the laser system of the first aspect, the first optical delivery element comprises a bundle of optical fibers and/or is configured to multiplex a plurality of the laser outputs of the laser source into one fiber at an input of the first optical delivery element.

This may improve a flexibility of the spatial modulation of the laser light as well as the scattered light detection mentioned above.

In a further implementation of the laser system of the first aspect, the detecting element may comprise a conductivity detecting element.

In a further implementation of the laser system of the first aspect, the conductivity detecting element may be configured to detect a conductivity on the tissue or on the object.

In some examples, it may be beneficial to form a stabilized porous structure. This can be realized through a stabilized gas bubbles generation. A spatial and/or temporal modulated laser light is capable of generating the microbubbles from the gas dissolved in a liquid in the environment. These bubbles may be stabilized by positive charges at their surfaces. The conductivity information may, therefore, reflect the status of the bubble formation. Modulating the laser light considering this information may facilitate a controlled generation of stabilized gas bubbles. It may further facilitate a controlled formation of the stabilized porous structure.

The characteristics of the porous structure, for example the pore width and length can be important parameters in optimizing one or more other processes, such as a laser treatment on a ciliary body, since these characteristics may reflect the overall mechanical property of the eye which may be used to optimize the other processes. Therefore, the controlled formation and an accurate detection of the corresponding characteristics can be important to achieve the effect of the one or more other processes. In particular, a real-time feedback controller may realize such controlled interaction between the porous structure formation and the one or more other processes.

In a further implementation of the laser system of the first aspect, the modulated laser light may be suitable for achieving and/or maintaining a first temperature range and/or a second temperature range in the first area, wherein the porous structure is stabilized in the first temperature range, and the porous structure is destabilized in the second temperature range.

For a stabilized porous structure formation, the parameters may be adjusted so that the detected temperature can be maintained below a first temperature threshold, and/or within the predetermined first temperature range. The gas bubbles can be stabilized in a lower temperature range. In an exemplary configuration, the first temperature threshold may be determined to be a value no smaller than 40 °C and/or no larger than 80 °C. In an example, the first temperature range may be 40 - 80 °C, in particular 45-65 °C.

In another example, the gas bubbles can be destabilized in a higher temperature range. The parameters may be therefore adjusted so that the detected temperature can be maintained above a second temperature threshold, and/or within the predetermined second temperature range to destabilize the porous structure. In an exemplary configuration, the second temperature threshold may be determined to be a value no smaller than 40 °C and/or no larger than 80 °C. In an example, the second temperature range may be 55 - 100 °C, preferably 70-90 °C.

In a further implementation of the laser system of the first aspect, the laser source may be regulated to form and/or stabilize one or more pores on the sclera, when the IOP of the eye needs to be reduced.

In a further implementation of the laser system of the first aspect, the laser source may be regulated to close and/or destabilize one or more pores on the sclera, when the IOP of the eye needs to be increased.

The present disclosure provides a laser device, which is able to reversibly change the IOP. This can be realized by opening and closing pores to increase and reduce the aqueous humor outflow depending on the desired effect. The opening and closing pores may be realized through the stabilizing and destabilizing the pores using a laser source. In other words, the solution provided by the present disclosure provides the opportunity to correct IOP change, which increases its flexibility, adaptability and preciseness of the IOP change.

The formation and/or destabilization of the one or more pores can be realized by regulating the laser source. For example, the laser source can be regulated such that in the first area a temperature can be maintained in the first temperature range and the porous structure can be stabilized, and/or the laser source can be regulated such that in the first area a temperature can be maintained in the second temperature ranges and the porous structure can be destabilized. The porous structure can also be stabilized/destabilized through other mechanism such as heating without laser.

In a further implementation of the laser system of the first aspect, the feedback controller may be configured to calculate a flow rate of fluid through the porous structure on the sclera based on the detected information.

The detected information may comprise the size distributions of pores, defects, or potential gas bubbles on the sclera as mentioned above. The calculation may be realized based on a hydrodynamic model constructed considering the mass transfer theory of fluid through the porous structure according to the detected information.

In a further implementation of the laser system of the first aspect, the second area may be outside the first area.

Through detecting the one or more physical, chemical, mechanical and/or structural characteristics in an area outside the first area, instead of only considering a primary effect of the laser light direct in the irradiated area, a secondary influence of the laser light on the surroundings may be considered as well. In addition, since the second area in this case is not influenced directly by the laser light, the noises in the detected information may be reduced. This may increase the precision of the feedback controller.

In a further implementation of the laser system of the first aspect, the second area may comprise two parts, each having a different distance to the first area, and the detected information may pertain to one or more physical, chemical, mechanical and/or structural characteristics of the two parts of the second area respectively.

For a more precise feedback control, the detecting element may detect characteristics of two parts, each having a different distance to the first area. For example, this may reflect a gradient of the characteristics.

In a further implementation of the laser system of the first aspect, the second area may comprise a part on the cornea.

For example, during the operation of the laser system, the laser light may not directly affect the cornea. Nonetheless, since cornea may be coupled to the other parts that are irradiated, for example, the sclera, ciliary body, the Schlemm's canal, and/or the TM, the laser effect on those parts may change the characteristics on the cornea.

In a further implementation of the laser system of the first aspect, the detected information may pertain to one or more mechanical characteristics of the cornea.

The cornea and it's mechanical coupling to the other parts of the eye is rigid. Therefore, the cornea may be sensitive to the mechanical changes of the other parts of the eye, and the mechanical response of the cornea on the mechanical change of the other parts of the eye may be fast. Thus, the information pertaining to the mechanical characteristics of the cornea may be a good input parameter for a precise feedback control.

In a further implementation of the laser system of the first aspect, the feedback controller may be configured to acquire an overall mechanical property of the eye, comprising a mechanical property of the trabecular meshwork, a mechanical property of a superficial layer of the Schlemm's canal, a mechanical property of a ciliary body and/or a mechanical property of the sclera.

For example, an overall mechanical property of the eye may comprise the IOP of the eye.

In a further implementation of the laser system of the first aspect, the overall mechanical property of the eye, the mechanical property of the trabecular meshwork, the mechanical property of the superficial layer of the Schlemm's canal, the mechanical property of a ciliary body and/or the mechanical property of the sclera may pertain to the mechanical response of the eye and/or the sclera to the modulated laser light.

In a further implementation of the laser system of the first aspect, the detecting element may comprise an OCE device and a pneumatic device combined with the OCE device, wherein the detecting element continuously measures the one or more mechanical characteristics of the cornea.

In a further implementation of the laser system of the first aspect, the OCE device continuously measures the one or more mechanical characteristics of the cornea.

Conventional pneumatic device for measuring the one or more mechanical characteristics of the cornea operates in a pulsed mode which is conducted before or after a laser treatment of the eye. The present disclosure provides a solution where the OCE may be used for measurements of the one or more mechanical characteristics of the cornea continuously, such that the pneumatic device may be operated in a continuous mode. For example, after a pneumatic pulse is applied on the cornea, an OCE measurement may be conducted on the cornea in the real-time during the laser irradiation. In another example, the OCE measurement may be conducted on the dent created by the pneumatic pulse. This way, the pneumatic measurement may be carried out during the laser treatment, facilitating a precise real-time feedback control.

In a further implementation of the laser system of the first aspect, the first area may comprise a part on a ciliary body of the eye, and wherein the laser source may be regulated to activate one or more cells on the ciliary body.

Conventional laser systems applied on the ciliary body for changing the IOP are usually used for cyclophotocoagulation treatment where the part of the ciliary body is coagulated in a destructive manner such that the aqueous humor production is reduced. This only applies for reducing the IOP, and is of a destructive nature. The present disclosure provides a laser system, where the ciliary body may be regenerated or healed through activating one or more cells on it through by the modulated laser light. Thus in this implementation, the IOP can also be increased, and the damage on the ciliary body can be reverted.

In a further implementation of the laser system of the first aspect, the modulated laser light may be suitable for generating a temperature and/or pressure condition to activate the one or more cells.

In a further implementation of the laser system of the first aspect, the temperature and/or pressure condition may be defined through a temperature range and/or pressure range.

Cells may be activated to differentiate or regenerate when put under a specific thermal and/or pressure condition. Such conditions can be realized through a modulated laser light. For example, by absorbing the laser energy, the temperature of the local tissues and/or fluid may increase. This may further result in a thermal pressure. By spatially and temporally modulating the laser light, a desired temperature and/or pressure condition for activation may be realized.

For example, a typical temperature on a ciliary body in a cyclophotocoagulation treatment may be larger than 55 °C, in particular in a range of 60-110 °C. A typical temperature range on the ciliary body for a cell activation may be in a range of 30°C to 70°C, for example, 40-60 °C, in particular 45-55 °C. A typical pressure range on the ciliary body for a cell activation may be smaller than 50 KPa for example, 3-30 KPa, in particular 5-25 KPa.

In a further implementation of the laser system of the first aspect, the modulated laser light may be suitable for generating a thermomechanical wave, which can propagate to a third area outside the first area, and the one or more cells may be in the third area.

The healing effect in the laser treatment or in each laser treatment session may be achieved by a localized temporally and spatially modulated laser light via activating the remote cell. In some examples, the localized laser light may be absorbed in a tissue region of the volume between 0.01 to 10 mm³, in particular, 0.1 to 1 mm³. A small irradiated area may reduce damage to the tissue due to direct laser irradiation and facilitate a more energy efficient and controllable modulation of the laser light. Although the directly irradiated area according to the present disclosure may be small in some examples, a large area can be treated by the laser-induced effects through generating a thermomechanical wave.

A controlled thermomechanical activation of the one or more cells may be realized through a stress wave (a wave due to an oscillating thermal mechanical property of a medium) arising due to a non-uniform heating waves arising, in turn, as a result of the laser-induced coordinated rotational oscillations of water electric dipoles. The stress wave may propagate to the one or more cells and activate the one or more cells through generating a specific thermal and/or mechanical condition for the one or more cells.

In a further implementation of the laser system of the first aspect, the feedback controller may be configured to regulate the dosimetry of the laser source, so that the generated modulated laser light changes a temperature and/or a pressure in the first area in a specific sequence and/or simultaneously.

In a further implementation of the laser system of the first aspect, the feedback controller may be configured to regulate the dosimetry of the laser source, so that the generated modulated laser light changes a temperature and/or a pressure field, in particular a temperature and/or pressure distribution in the first area in a specific sequence and/or simultaneously.

Changing a temperature and/or a pressure in the first area in a specific sequence can facilitate a generation of the stress wave.

In an example, the temperature and/or pressure where the stress wave is generated can be changed and controlled by the modulated laser light according to the desired reachable range of the stress wave, and a behavior of the stress wave dissipation in the environment on the eye.

Changing a temperature and/or a pressure in the first area in a specific sequence and/or simultaneously can also facilitate a controlled formation of porous structure.

In a further implementation of the laser system of the first aspect, the feedback controller may be configured to control in a real-time, based on the real-time detected information, a position of the first optical delivery element during the irradiation.

For example, the feedback controller may be configured to control the first optical delivery element to guide the laser light to a fourth area different from the first area in a real-time based on the real-time detected information.

The first optical delivery element may comprise a single or a bundle of optic fibers. The first optical delivery element may comprise a plurality of outcoupling elements. The change of the irradiated area may comprise a switching between different outcoupling elements and/or controlling individual outcoupling elements, such as tilting an angle of the outcoupling element. The first optical delivery element may comprise a servo element, configured to change the physical location of the first optical delivery element for changing the irradiated area. The irradiated area may be changed after an activation of a cell and/or formation of a porous structure, to activate another cell and/or to foam another porous structure. The changing of the irradiated area may also be performed while activating the same cell or forming the same porous structure. In the latter case, the change of the irradiated area, as a part of the spatial modulation of the laser light, may facilitate a generation of a desired stress wave.

In a further implementation of the laser system of the first aspect, the first area may comprise at least two of the following:
a) a part on a sclera of the eye;
b) a part at and/or in the vicinity of a Schlemm canal and/or a trabecular meshwork of the eye; and
c) a part on the ciliary body of the eye,

The laser system according to the present disclosure may apply laser effect to different areas or parts of the areas on the eye during a laser treatment. The laser effects may occur at the same time or close to each other spatially or temporally, such that the final effect on a part on the eye may be complicated. For example, a final effect on a part on the eye may comprise a superposition of a primary effect of the laser light directly delivered to this part and a secondary effect of the laser light delivered to another part on the eye or a superposition of two secondary effects of laser light delivered to two other parts on the eye. The controller may regulate the laser source in a way such that the laser light delivered to the different parts on the eye can be modulated at the same time for a global optimal healing effect.

Among the different parts on the eye, the three kinds a) to c) mentioned above may be important for the IOP. A laser treatment considering at least two of the three parts, or all the three parts at the same time may result in an improved healing effect compared to the conventional laser treatment, where usually only the b) or c) is treated.

In a further implementation of the laser system of the first aspect, the feedback controller may be configured to calculate a desired relative contribution of the two parts on normalizing the IOP.

In a further implementation of the laser system of the first aspect, the dosimetry of the laser source may be regulated to achieve the desired relative contribution.

In an example, the relative contribution may be a relative contribution in an increased/reduced aqueous humor outflow and/or production.

Considering multiple parts on the eye at the same time may not only contribute to a precise determination of the real-time laser effect on these parts, but also contribute to a precise desired result determination. By achieving the final desired result considering multiple parts on the eye, each part on the eye may require less modification compared to only treating one of the parts, which may result in an overall less trauma.

For example, in a first laser treatment session, to reduce the IOP of an eye, a porous structure may be formed and stabilized in a part on the sclera of the eye using a first modulated laser light while the lumen area of the Schlemm's canal of the eye in another part may be increased using a second modulated laser light. The relative contribution of the two parts on normalizing the IOP may be 1:1. In an example, this value may be calculated through dividing the increased aqueous humor outflow through the uveoscleral pathway by the increased aqueous humor outflow through the conventional pathways.

For another example, some time after the first laser treatment session, it may be determined that the IOP of the eye may be too low and a second laser session may be necessary. In the second laser session, in a part on the sclera a porous structure formed and stabilized in the first laser session may be destabilized and closed using a third laser light while in a part on the ciliary body, one or more cells may be healed and activated using a fourth laser light. The relative contribution of the two parts on normalizing the IOP may be 1:2. In an example, this value may be calculated through dividing the reduced aqueous humor outflow through the uveoscleral pathway by the increased aqueous humor production by the ciliary body.

For another example, a desired relative contribution of three, more, or all parts on normalizing the IOP may be calculated. For example, a laser treatment session may involve applying laser effects on 3 parts on the sclera and 2 parts on the ciliary body, and their respective contribution on normalizing the IOP may be 25 %, 25 %, 30 %, 10 % and 10 % respectively.

In a further implementation of the laser system of the first aspect, the feedback controller may be configured to determine, based on the detected information, whether one of the parts has been treated before.

A part on the eye that has been treated before may be sensitive to further laser effect and the further laser effect may result in more trauma in such part than other parts. Through determining whether one of the parts has been treated before, the controller may further be configured to regulate laser source accordingly to reduce the real-time laser effect on this treated part or to reduce an overall laser dose applied in this treated part.

In a further implementation of the laser system of the first aspect, when the feedback controller determines that the one of the parts has been treated before, the feedback controller is configured to correct the desired relative contribution by reducing the desired contribution of the treated part.

Such correction can be realized based on multiplying the desired contribution with a predetermined factor. For example, a predetermined factor may be 0.25. A desired relative contribution of a part on the sclera and a part on the TM on reducing the IOP may be 1:1 before a correction. It may be determined that the TM has been treated before, for example, through a conventional selective laser trabeculoplasty. The desired relative contribution after the correction may be then 1:0.25 = 4:1. If these are the only two parts irradiated by laser lights, this means that the laser system may operate in a way that after the laser treatment session, 80 % instead of 50 % of the increased aqueous humor outflow may be realized through the porous structure newly formed in the part on the sclera, while 20 % instead of 50 % of the increased aqueous humor outflow may be realized through the laser effect on the part at the TM.

In an example, the factor may depend on which part has been treated before, the result of the previous treatment, other patient related features and/or a diagnosis.

In an example, desired contributions of more than one part may be corrected.

In a further implementation of the laser system of the first aspect, the two or more parts may be irradiated simultaneously, sequentially and/or repeatedly.

In a further implementation of the laser system of the first aspect, the first area may comprise a plurality of parts arranged along a limbus rim of the eye.

By arranging a plurality of parts along the limbus rim, the treatment may be more symmetrical and hence more balanced.

In an example, a plurality of parts on the same organ on the eye, for example a plurality of parts on the sclera, may be irradiated with laser light stemming from the same laser source.

In another example, the plurality of parts on the eye may be irradiated with laser lights that are correlated to each other. For example, the laser lights irradiating adjacent areas on the eye may have a certain phase shift.

In a further implementation of the laser system of the first aspect, the feedback controller may comprise and/or be coupled to a (remote) high-performance computer, a (remote) hybrid quantum-classical computational facility, and/or a (remote) quantum computer.

In a further implementation of the system of the first aspect, the feedback controller may comprise and/or be connected to a storage device, the storage device storing an offline settings table, wherein the settings table is calculated by a (remote) high-performance computer, a (remote) hybrid quantum-classical computational facility, and/or a (remote) quantum computer.

The real-time regulation of the laser based on feedback detected information of the laser affected area according to the present disclosure is a complex feedback optimization problem. A better evaluation of the laser effect and a precise regulation of the laser may rely on a large volume of the detected information, which may be an information of a huge volume. Quantum algorithms or hybrid-quantum algorithms such as a variational quantum eigensolvers may be employed in this context and may outperform a conventional algorithm in optimizing a system with parameters of multiple dimensions. Therefore, using a high-performance, and /or hybrid-, and/or quantum-computer, and/or a hybrid computational facility may facilitate a better control over the laser system.

In a further implementation of the method of the first aspect, the remote high-performance computer, the remote hybrid quantum-classical computational facility, and/or the remote quantum computer may be located in a central server.

In a further implementation of the method of the first aspect, the central server may be configured to regulate a plurality of laser systems.

A second aspect of the disclosure provides a method, comprising:
a) detecting one or more physical, chemical, mechanical and/or structural characteristics in a first area on an eye;
b) processing the detected information pertaining to the physical, chemical, mechanical and/or structural characteristics in the first area on the eye; and
c) acquiring a property of a porous structure change on the eye and/or an activation of a ciliary body regeneration of the eye, in a real-time during the porous structure change and/or the activation of ciliary body regeneration.

In an implementation of the method of the second aspect, the property of the porous structure change and/or the activation of a ciliary body regeneration may comprise a real-time aqueous humor outflow calculated based on the porous structure change and/or a real-time aqueous humor production calculated based on the generation of the ciliary body.

In a further implementation of the method of the second aspect, the characteristics may comprise one or more of the following: IOP of the eye; a temperature; a temperature distribution; a pressure; a pressure distribution; a Young's modulus; a speed of sound; a chemical composition; a thickness, for example a thickness of sclera; a dimension, for example a dimension of ciliary body, Schlemm's canal and/or TM; a dimension of an unblocked lumen area, for example, a dimension of an unblocked lumen area in a humor drain pathway; a pore size; a pore size distribution; a characteristics of a scattered light.

In a further implementation of the method of the second aspect, the method steps may be carried out in a real-time during a laser irradiation on a second area of the eye.

The laser light used in the irradiation may exert an invasive effect on the second area, for example, in a laser treatment. It may also not exert any essential invasive effect on the second area, for example, for a diagnosis. For example, the laser light in the irradiation may be a probing light used for light scattering analysis. In another example, the light scattering analysis may be used to acquire a property of a porous structure.

In a further implementation of the method of the second aspect, the information detected and processed may be used to regulate a laser source in a real-time for optimizing the porous structure change and/or the ciliary body regeneration.

Although the present disclosure may provide an automatic feedback-control laser system, for example the laser system according to the first aspect, it is understood that the method according to the second aspect need not encompass the regulation of laser system itself. For example, the method according to the second aspect can provide the doctor or the practitioner operating a laser with the necessary information based on which the doctor or the practitioner may subsequently evaluate the aqueous humor outflow/production in the first area on the eye and/or an expected laser effect in this area.

An evaluation system, configured to perform the method according to the second aspect may comprise an indicator, for example an indicating LED light bulb.

In an example, if the evaluation system determines that an aqueous humor outflow/production of the area needs to be changed, it may indicate to the doctor or the practitioner to perform a laser treatment, for example, through showing a green light. It may further indicate the doctor or the practitioner where to perform a laser treatment, for example through showing the corresponding information on a screen.

In another example, if the evaluation system determines that the detected information on the to-be treated eye reaches a predetermined value, for example, if a porosity of a porous structure on the eye is large enough, or a temperature is too high, it may indicate to the doctor or the practitioner to stop the laser treatment, for example through showing a red light.

The method may also provide indications to the doctor to perform other actions, for example to change the dosimetry of the laser. The predetermined value and/or other evaluation criteria may be predetermined by the doctor, or the practitioner based on concrete cases, or stored in an offline settings table for the treatment, wherein the setting table may be calculated by a remote high-performance computer, a remote hybrid quantum-classical computational facility, and/or a remote quantum computer. The predetermined value and/or other evaluation criteria may be predetermined based on the laser used by the doctor or the practitioner, which may be a laser in the laser system according to the first aspect of the disclosure. The laser may also be a laser where the dosimetry can be adjusted manually.

In a further implementation of the method of the second aspect, the processing the detected information may comprise: generating a value for a dosimetry of a laser source in a real-time during the porous structure change and/or the activation of the ciliary body regeneration based on the detected information.

In an automatic laser system such as the laser system according to the first aspect of the disclosure, the generated value for a dosimetry of the laser may be directly used by the feedback controller to regulate the laser dosimetry without a human interference. The value may also be displaced to the doctor or the practitioner, so that they can use it to manually adjust a laser dosimetry or to decide whether to stop the laser treatment. As long as the detecting and the processing of the information can be performed in a real-time, for example within several minutes, the doctor or the practitioner may have enough time to react to change the laser dosimetry in time for a real-time laser effect, even if the doctor or the practitioner chooses to change the laser dosimetry manually. Compared to the conventional monitoring and evaluation systems, an evaluation system adopting the method according to the second aspect provides a more informative and precise feedback to the doctor or the practitioner operating a laser system for treating an eye with abnormal IOP.

In a further implementation of the method of the second aspect, the detecting of the physical, chemical, mechanical and/or structural characteristics in the area may comprise: determining a temperature or a temperature field in the area, wherein the value for the dosimetry of the laser source is generated if the temperature and/or its distribution is within a predetermined range.

In a further implementation of the method of the second aspect, the value for the dosimetry of the laser source may not be generated if the temperature is not within the predetermined range.

In a further implementation of the method of the second aspect, the processing the detected information may be conducted according to the current task to provide the practitioner or the doctor the supplementary information on how to operate the laser system.

The task may be one of: a change of porous structure on the sclera; a change of the lumen area at and/or in the vicinity of a Schlemm canal and/or a trabecular meshwork; a ciliary body coagulation; and a ciliary body regeneration.

In a further implementation of the method of the second aspect, the processing of the detected information pertaining to the physical, chemical, mechanical and/or structural characteristics on the eye may further comprise a consideration of the effect of detected temperature on the thermodynamic parameters of the medium, in particular, on the nonlinearity of the thermodynamic parameters.

Laser lights during the laser treatment may be absorbed by the medium. Most modern diagnostic techniques based on a laser treatment ignores the effect of the local temperature increase of the medium on the thermodynamic parameters of the medium (e.g., thermal conductivity, density, thermal-expansion coefficient, and isobaric specific heat capacity) due to such laser light absorption. The conventional diagnostic techniques assume that the thermodynamic parameters are constant. However, even a small increase in local temperature can change the values of thermodynamic parameters of the medium, and it can be necessary to consider the nonlinearity of the thermal parameters in the thermal diffusion thermo-mechanical equations. Most serious alterations in the parameters can be due to structural and phase transformation occurring in the tissue during laser irradiation.

In a further implementation of the method of the second aspect, the first area may comprise a part outside the second area.

In a further implementation of the method of the second aspect, the second area may be irradiated by a spatial and/or temporal modulated laser light generated by the laser source.

In a further implementation of the method of the second aspect, the first area may comprise two parts, each having a different distance to a second area, wherein the detected information may pertain to one or more physical, chemical, mechanical and/or structural characteristics of the two parts of the first area respectively.

In a further implementation of the method of the second aspect, a detected pressure distribution may be mapped onto a detected temperature distribution in the first area.

A spatially resolved distribution may provide more information about the laser effect, which may increase the precision of the laser regulation.

The combination of the temperature detection and a mechanical pressure detection may reflect different properties in the area on the eye and may facilitate a precise evaluation of the laser effect.

In a further implementation of the method of the second aspect, the first area may comprise a part on a cornea of the eye.

In a further implementation of the method of the second aspect, the detected information may pertain to the mechanical characteristics of the cornea.

In a further implementation of the method of the second aspect, the acquiring the property of a porous structure change on the eye and/or an activation of a ciliary body regeneration of the eye, in a real-time during the porous structure change and/or the activation of ciliary body regeneration may further comprise: acquiring an overall mechanical property of the eye, comprising a mechanical property of the trabecular meshwork, a mechanical property of a superficial layer of the Schlemm's canal, a mechanical property of a ciliary body and/or a mechanical property of the sclera.

In a further implementation of the method of the second aspect, the overall mechanical property of the eye, the mechanical property of the trabecular meshwork, the mechanical property of the superficial layer of the Schlemm's canal, the mechanical property of a ciliary body and/or the mechanical property of the sclera may pertain to the mechanical response of the eye and/or the sclera to a spatial/temporal modulated laser light.

In a further implementation of the method of the second aspect, the processing of the detected information may be performed in a (built-in or remote) high-performance computer, a (built-in or remote) hybrid quantum-classical computational facility, and/or a (built-in or remote) quantum computer.

In a further implementation of the method of the second aspect, the method of the second aspect may be encompassed in an algorithm designed for the high-performance computer, the hybrid quantum-classical computational facility, and/or the quantum computer.

In a further implementation of the method of the second aspect, the remote high-performance computer, the remote hybrid quantum-classical computational facility, and/or the remote quantum computer may be located in a central server.

In a further implementation of the method of the second aspect, the central server may be configured to regulate a plurality of laser systems.

A third aspect of the disclosure provides a method for changing an IOP of an eye using a temporal and/or spatial modulated laser light comprising a treatment step, wherein the treatment step comprises:
a) detecting one or more physical, chemical, mechanical and/or structural characteristics in a first area on the eye in a real-time during the change of the IOP; and
b) modulating the laser light irradiating a second part of the area in a real-time based on the real-time detected information.

In an implementation of the method of the third aspect, the characteristics may comprise one or more of the following: IOP of the eye; a temperature; a temperature distribution; a pressure; a pressure distribution; a Young's modulus; a speed of sound; a chemical composition; a thickness, for example a thickness of sclera; a dimension, for example a dimension of ciliary body, Schlemm's canal and/or TM; a dimension of an unblocked lumen area, for example, a dimension of an unblocked lumen area in a humor drain pathway; a pore size; a pore size distribution.

In a further implementation of the method of the third aspect, the second area may comprise a part on a sclera of the eye.

In a further implementation of the method of the third aspect, the laser light may be modulated to modify a porous structure on the sclera.

In a further implementation of the method of the third aspect, the modulated laser light may be suitable for achieving and/or maintaining a first temperature range and/or a second temperature range in the second area, wherein the porous structure is stabilized in the first temperature range, and the porous structure is destabilized in the second temperature range.

In a further implementation of the method of the third aspect, the laser light may be modulated to form and/or stabilize one or more pores on the sclera, when the IOP of the eye needs to be reduced.

In a further implementation of the method of the third aspect, the laser light may be modulated to close and/or destabilize one or more pores on the sclera, when the IOP of the eye needs to be increased.

In a further implementation of the method of the third aspect, the method may further comprise: calculating a flow rate of fluid through the porous structure on the sclera based on the detected information.

In a further implementation of the method of the third aspect, the first area may be outside the second area.

In a further implementation of the method of the third aspect, the first area may comprise two parts, each having a different distance to the second area, wherein the detected information may pertain to one or more physical, chemical, mechanical and/or structural characteristics in the two parts of the first area respectively.

In a further implementation of the method of the third aspect, the first area may comprise a part on the cornea.

In a further implementation of the method of the third aspect, the detected information may pertain to one or more mechanical characteristics of the cornea.

In a further implementation of the method of the third aspect, the method may further comprise: acquiring an overall mechanical property of the eye, comprising a mechanical property of the trabecular meshwork, a mechanical property of a superficial layer of the Schlemm's canal, a mechanical property of a ciliary body and/or a mechanical property of the sclera.

In a further implementation of the method of the third aspect, the overall mechanical property of the eye, the mechanical property of the trabecular meshwork, the mechanical property of the superficial layer of the Schlemm's canal, the mechanical property of a ciliary body and/or the mechanical property of the sclera may pertain to the mechanical response of the eye and/or the sclera to the modulated laser light.

In a further implementation of the method of the third aspect, the second area may comprise a part on a ciliary body of the eye.

In a further implementation of the method of the third aspect, the laser source may be regulated to activate one or more cells on the ciliary body.

In a further implementation of the method of the third aspect, the modulated laser light may be suitable for generating a temperature and/or pressure condition to activate the one or more cells.

In a further implementation of the method of the third aspect, the modulated laser light may be suitable for generating a thermomechanical wave, which can propagate to a third area outside the second area, wherein the one or more cells may be in the third area.

In a further implementation of the method of the third aspect, the second area may comprise at least two of the following:
a) a part on a sclera of the eye;
b) a part at and/or in the vicinity of a Schlemm canal and/or a trabecular meshwork of the eye; and
c) a part on the ciliary body of the eye,

In a further implementation of the method of the third aspect, the feedback controller may be configured to calculate a desired relative contribution of the two parts on normalizing the IOP.

In a further implementation of the method of the third aspect, the laser light may be modulated to achieve the desired relative contribution.

In a further implementation of the method of the third aspect, the method may further comprise: determining, based on the detected information, whether one of the parts has been treated before.

In a further implementation of the method of the third aspect, when the feedback controller determines that the one of the parts has been treated before, the desired relative contribution may be corrected by reducing the desired contribution of the treated part.

In a further implementation of the method of the third aspect, the two parts may be irradiated simultaneously, sequentially and/or repeatedly.

In a further implementation of the method of the third aspect, the second area may comprise a plurality of parts arranged along a limbus rim of the eye.

In a further implementation of the method of the third aspect, the method may be carried out by a high-performance computer, a hybrid quantum-classical computational facility, and/or a quantum computer.

In a further implementation of the method of the third aspect, the method may be carried out by a feedback controller which comprises and/or is connected to a storage device, the storage device storing an offline settings table, wherein the settings table is calculated by a high-performance computer, a hybrid quantum-classical computational facility, and/or a quantum computer.

### BRIEF DESCRIPTION OF THE DRAWINGS

To illustrate the technical features of embodiments of the present disclosure more clearly, the accompanying drawings describing the embodiments are introduced briefly in the following description. The accompanying drawings in the following description are merely some embodiments of the present disclosure, and modifications of these embodiments are possible without departing from the scope of the present disclosure as defined in the claims.
- FIG. 1: is a schematic illustration of an anterior section of an eye,
- FIG. 2: is a schematic illustration of a laser system according to an embodiment,
- FIG. 3: is a schematic illustration of a laser system according to an embodiment,
- FIG. 4: is a flow chart illustrating a method for detecting and processing information according to an embodiment,
- FIG. 5: is a flow chart illustrating a method for changing an IOP of an eye using a temporal and/or spatial modulated laser light,
- FIG. 6: is a schematic illustration of an eye under a laser treatment or diagnosis according to an embodiment,
- FIG. 7: is an example of the calculated size distribution of pores in trabecular meshwork as a result of laser irradiation of a rabbit eye.
- FIG. 8: is a graphical representation of a calculated kinetic of the porous structure formation in a laser treated sclera of a human eye using a laser.
- FIG. 9: is a histological image of laser-induced pores in sclera of a rabbit eye.
- FIG. 10: is an ultrasound image of an irradiated sclera of a rabbit eye.
- FIG. 11: is a graphical representation of the calculated spatial distribution of temperature in trabecular meshwork during laser irradiation of a rabbit eye.
- FIG. 12: is a histological image of laser-induced coagulation of ciliary body of a rabbit eye.
- FIG. 13: is a graphical representation of the measured dynamic of temperature and backscattered light during laser irradiation of a sclera of a human eye.

### DETAILED DESCRIPTION OF FIGURES AND EXAMPLES

The following description presents examples of the implementation of the present disclosure, and the scope of the present disclosure, but the disclosure is not limited to presented examples. Any variations or replacements can be easily made by persons skilled in the art. Accordingly, the scope of protection of the present disclosure is defined by the attached claims.

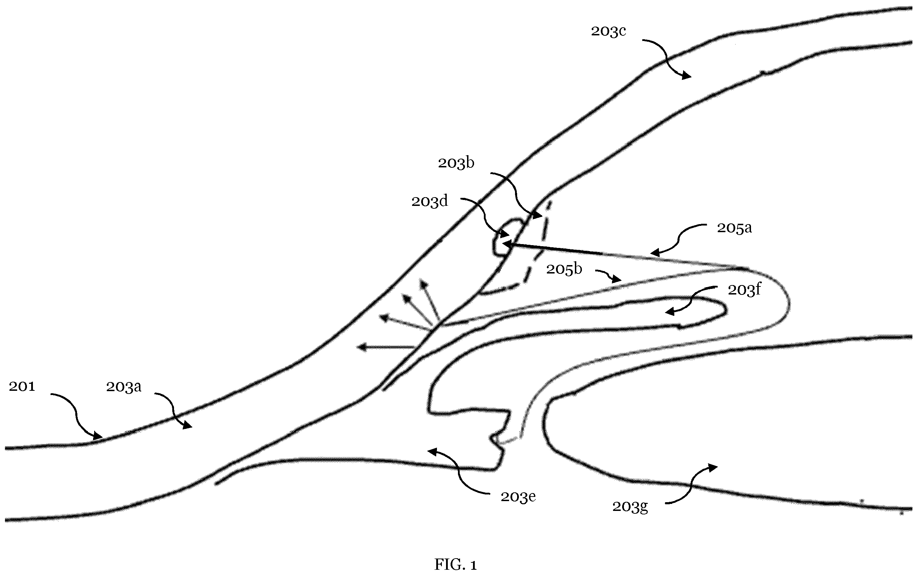

FIG. 1 is a schematic illustration of an anterior section of an eye 201. In the anterior section, multiple organs, which are relevant for an intraocular pressure, IOP, of the eye 201 are located in or near the limbus rim which surrounds the outer edge of the iris 203f or the outer edge of the lens 203g, where the sclera 203a meets the cornea 203c. The IOP is kept in balance through the production and outflow of the aqueous humor. The aqueous humor is produced in the ciliary body 203e and is drained following two typical outflow pathways: a conventional pathway 205a or an uveoscleral pathway 205b. In both types of outflow pathways 205a and 205b, the aqueous humor produced by the ciliary body 203e flows through the posterior chamber between the iris 203f, the ciliary body 203e and the lens 203g, then through the anterior chamber between the iris 203f, lens 203g and the cornea 203c, and then towards the limbus rim area of the anterior chamber. In a conventional pathway 205a, the aqueous humor is finally drained through the trabecular meshwork 203b and the Schlemm's canal 203d. In an uveoscleral pathway 205b, instead of drained through the trabecular meshwork 203b and the Schlemm's canal 203d, the aqueous humor is finally drained through the sclera 203a, which is located in the vicinity of or apart from the trabecular meshwork 203b or the Schlemm's canal 203d.

In the eyes of human infants, the sclera 203a is porous and the uveoscleral pathway 205b contributes significantly to the total drain of the aqueous humor. In the eyes of a human adult, the pores in the sclera 203a close up and the contribution of the uveoscleral pathway 205b to the total drain of the aqueous humor drops significantly.

### Exemplary System

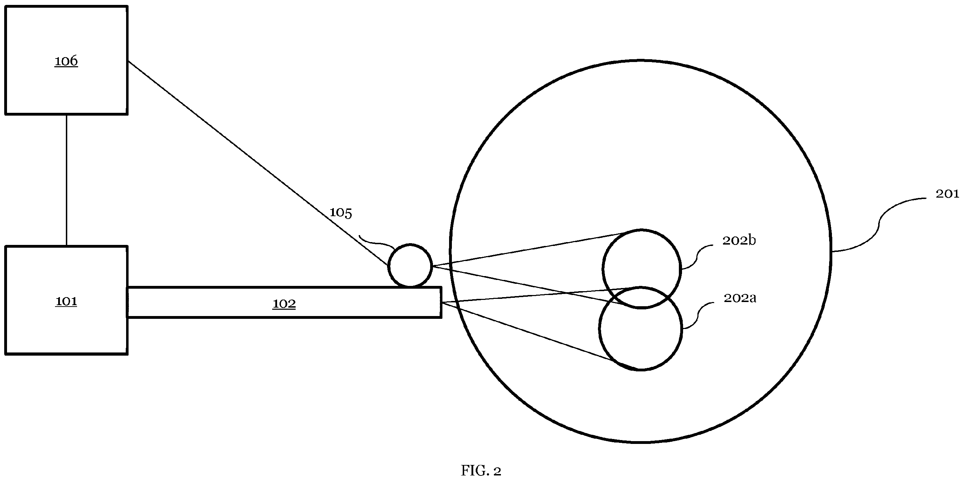

FIG. 2 is a schematic illustration of a laser system disclosed by the present disclosure.

The laser system is suitable for changing an IOP of an eye 201. The laser system comprises: a laser source 101; a feedback controller 106, configured to regulate a dosimetry of the laser source 101 to produce spatially and/or temporally modulated laser light; a first optical delivery element 102, configured to guide the spatially and/or temporally modulated laser light to irradiate a first area 202a on the eye 201; and a detecting element 105, configured to detect one or more physical, chemical, mechanical and/or structural characteristics in a second area 202b on the eye 201 in a real-time during the change of the IOP, wherein the feedback controller 106 is configured to regulate the dosimetry of the laser source 101 in a real-time based on the real-time detected information pertaining to the one or more physical, chemical, mechanical and/or structural characteristics in the second area 202b.

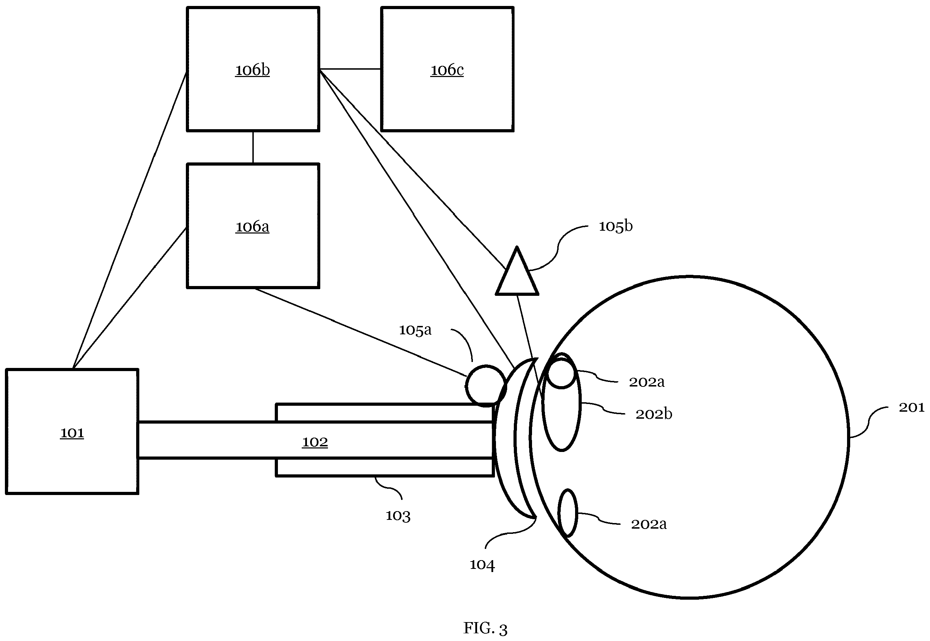

FIG. 3 is a schematic illustration of a laser system according to an embodiment.

The laser system may comprise an electronic unit 106a. The electronic unit 106a may comprise a diagnostic element, configured to receive a detected information and process the detected information. The diagnostic element comprises a User Interface, configured to present the detected information to a user, e.g., a researcher or a medical doctor. For example, the User Interface may be configured to present a stress distribution and a temperature distribution in the area 202b. The diagnostic element may send the detected information unprocessed to a remote high-performance computer, the remote hybrid computational facility, and/or the remote quantum computer 106c. The diagnostic element may further be configured to preprocess the detected information. For example, the diagnostic element may be configured to analyze a detected information pertaining to a scattered light and determine a size distribution of pores in a porous structure.

The electronic unit 106a may comprise a radiation modulation element, configured to modulate the radiation of the laser 101 source temporally and spatially. The radiation modulation element may be configured to receive a command generated for modulating the radiation of the laser source 101 and regulate a dosimetry of the laser source 101, or the radiation modulation element may be configured to receive the dosimetry value directly from the external high-performance computer, the remote hybrid computational facility, and/or the remote quantum computer 106c.

The laser system may further comprise a feedback control element 106b, configured to manage a data flow in the laser system. The data flow may comprise a flow of real-time detected information pertaining to one or more physical, chemical, mechanical and/or structural characteristics in the area 202b; a flow of processed/preprocessed detected information; a command generated for regulating a dosimetry of a laser source 101. The feedback control element 106b may be configured to control the direction and a sequence of the data flow, so that the irradiation of the laser source 101 can be modulated in a real-time based on real-time detected information.

The laser system may further comprise an external high-performance computer, a remote hybrid computational facility, and/or a remote quantum computer 106c configured to process the detected information or the preprocessed detected information to generate a command for modulating the radiation of the laser source 101 or to regulate a dosimetry of the laser source 101.

The external high-performance computer, the remote hybrid computational facility, and/or the remote quantum computer 106c may be configured to solve equations defining a thermal mechanical problem such as a heat propagation problem, a mechanical problem such as a problem regarding a medium deformation. The solution may help to optimize a control of a porous structure changing and/or cell activation.

The external high-performance computer, the remote hybrid computational facility, and/or the remote quantum computer 106c may be configured to solve equations defining a chemical process problem such as a chemical bond breaking problem.

The external high-performance computer, the remote hybrid computational facility, and/or the remote quantum computer 106c may be configured to calculate the dynamics of pore shape and size. The solution may help to optimize a controlled formation of porous structure.

The external high-performance computer, the remote hybrid computational facility, and/or the remote quantum computer 106c may be configured to solve equations defining a hydrodynamic problem, for example the mass transfer of aqueous humor through a porous structure. The solution may help to control the real-time IOP and to optimize the sensibility of the feedback control of the laser source.

The external high-performance computer, the remote hybrid computational facility, and/or the remote quantum computer 106c may be configured to use the solution of the abovementioned inverse problem to establish optimal dosimetry for each step of laser treatment. The calculations may be conducted within a small time interval, for example, within a millisecond up to several minutes, so that the method for treating the eye 201 according to the present disclosure can be carried out continuously.

The diagnostic element, the feedback control element 106b, the radiation modulation element and the high-performance computer, the remote hybrid computational facility, and/or the remote quantum computer 106c may be parts of the feedback controller 106 in FIG.1. Although FIG. 2 shows a separation of the electronic unit 106a, the feedback control element 106b and the high-performance computer, the remote hybrid computational facility, and/or the remote quantum computer 106c, this separation should not be interpreted as a physical separation but rather a separation of their logical functions. The feedback controller 106 may also refer to a combination of one or more of the diagnostic elements, the feedback control element 106b, the radiation modulation element and the high-performance computer, the remote hybrid computational facility, and/or the remote quantum computer 106c.

For example, if the feedback controller 106 is only configured to process the detected information pertaining to a physical, chemical, mechanical and/or structural characteristics in the area 202b, acquiring a property of a porous structure change on the eye 201 and/or an activation of a ciliary body regeneration of the eye 201, the diagnostic element alone or a combination of the diagnostic element and the high-performance computer, the remote hybrid computational facility, and/or the remote quantum computer 106c can be seen as a feedback controller 106, then, in this case, the feedback controller 106 facilitates an evaluation of the porous structure change on a sclera of the eye 201 and/or the activation of a ciliary body regeneration of the eye 201 in the area 202b and an initialization of parameters for the laser source 101.

For example, if the feedback controller 106 is further configured to process the detected information in a real-time during the porous structure change and/or the activation of the ciliary body regeneration induced by the temporally and spatially modulated radiation of the laser source 101, a combination of the feedback control element 106b and the diagnostic element 106a can be seen as a feedback controller 106, then, in this case, the feedback controller 106 facilitates a monitoring of the laser-induced porous structure change and/or the activation of the ciliary body regeneration. For example, a doctor can decide on their own when to interrupt the laser irradiation depending on whether the size distribution of pores in the porous structure reaches a predetermined threshold.

The feedback controller 106 may be configured to regulate in a real-time the laser source 101 based both on a diagnostic data obtained prior to a laser treatment session and on the real-time-detected information during the laser treatment session.

The laser system may comprise a laser 101, configured in a way that its radiation is spatially and temporally modulated by the feedback controller 106. Spatial modulation may refer to varying a location, a shape of the laser beam and the laser-illuminated area and a certain intensity distribution of the laser-induced light in the laser illuminated area. To realize such a spatial modulation, the laser system may comprise one or more lasers sources 101. The laser light delivered by the laser source 101 may be both coherent and non-coherent. A plurality of lasers 101 may facilitate a complicated spatial modulation of laser irradiation.

The laser system may comprise an optical converting element 104. The optical converting element 104 may comprise active or passive elements, such as LEDs, lasers, lenses, mirrors, an optical splitter, and other optical systems thereof. The optical converting element may facilitate or supplement a spatial modulation. In an example, the converting element is suitable to guide the modulated laser light to a plurality of parts aligned along the limbus rim.

Each one of the lasers 101 may implement an independent temporally modulated irradiation. A temporally modulated laser irradiation is usually a sequence of pulses of laser irradiation with variable pulse repetition rate, pulse duration, pulse intensities or other variable attributes of a laser pulse. A temporal modulated laser radiation may also refer to non-pulsed laser radiation with a variable shape in the time domain and a variable shape in the frequency domain.

The irradiation of the laser sources 101 may be real-time modulated. A real-time modulation may include a constantly regulating a dosimetry of the laser source 101, regulating the dosimetry upon receiving a signal from the feedback controller 106 or updating the laser dosimetry after a certain number of pulses in a sequence.

A laser source 101 in the present disclosure may be a combination of several types of lasers, including a solid-state laser, a fiber laser and/or a diode laser.

Each of the laser sources 101 may further be assigned to different tasks. For example, during a treatment, a first laser source 101 may generate modulated laser light to close pores on the sclera, while a second laser source 101 may generate a modulated laser light to activate the ciliary body regeneration.

The laser system may further comprise one or more optical delivery elements 102, configured to deliver the modulated laser radiation or laser light to a target. The optical delivery elements 102 can be an optical fiber, a bundle of optical fibers, or other types of optical delivery elements. The optical delivery elements 102 may also be configured to deliver other laser signals, for example, a probing laser signal for detecting a certain property on the eye 201. In an exemplary embodiment, the imposed laser modulation may account for the possible distortion of the laser signal due to propagation in the laser delivery system 102 and implement corresponding compensations. The optical delivery element 102 may comprise an optical out-coupler for delivering the laser signals in a form of laser irradiation to the target. The out-coupler may be coupled with the converting element 104.

The laser system may further comprise one or more detecting elements 105. The detecting elements 105 are configured to detect one or more physical, chemical, mechanical and/or structural characteristics in the area 202b on the eye 201. The one or more physical, chemical, mechanical and/or structural characteristics may comprise one or more of the following: IOP of the eye; a temperature; a temperature distribution; a pressure; a pressure distribution; a Young's modulus; a speed of sound; a chemical composition; a thickness, for example a thickness of sclera; a dimension, for example a dimension of ciliary body, Schlemm's canal and/or TM; a dimension of an unblocked lumen area, for example, a dimension of an unblocked lumen area in a humor drain pathway; a pore size; a pore size distribution.

The characteristics may be detected in a direct and in an indirect way. In an example, the detecting element 105 may comprise an optical receiving element, configured to receive a scattered light. The scattered light can be feedbacked as an optical signal and be processed to deliver the information about a temperature, a pressure, a size distribution of gas bubbles, and a size distribution of pores and other structural defects, based on the characteristics of the optical signal such as, for example but not restricted to, wavelength distribution and an angular intensity distribution. In an exemplary embodiment, the detecting element 105 may comprise a diagnostic device, such as one of the following: an IR radiometry; a photoacoustic detector; an OCE device; an OCT device; and a device for detecting backlight scattering.

The detecting element 105 may further comprise a pneumatic device for supplying an air pulse and/or controlling the air pressure. In an example, the pneumatic element may be used for IOP measurement. In another example, the pneumatic element may be used combined with OCE measurements of mechanical properties, such as Young's modulus and a pressure distribution on cornea, sclera and at or in the vicinity of trabecular meshwork. In another example, the mechanical properties can be measured in a continuous mode in a real-time during a laser treatment.

The laser system may comprise a working tool 103. The working tool may be connected to the laser source 101 through the one or more optical delivery elements 102. Part of the optical delivery elements 102, one or more detecting element 105, the optical converting element 104, and/or (part of) the electronic unit 106a may be incorporated with or integrated on the working tool 103. The working tool 103 may be further connected to the feedback control element 106b.

### Exemplary method

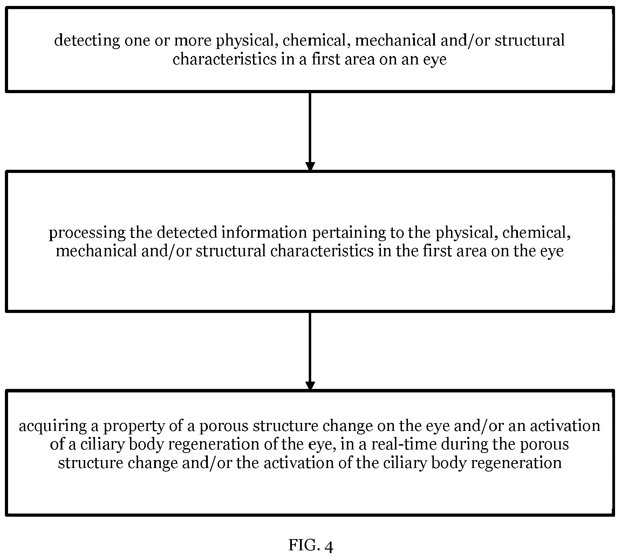

FIG. 4 is a flow chart illustrating a method for detecting and processing information according to an embodiment.

In this embodiment, the method comprises:
a) detecting one or more physical, chemical, mechanical and/or structural characteristics in a first area on an eye;
b) processing the detected information pertaining to the physical, chemical, mechanical and/or structural characteristics in the first area on the eye; and
c) acquiring a property of a porous structure change on the eye and/or an activation of a ciliary body regeneration of the eye, in a real-time during the porous structure change and/or the activation of the ciliary body regeneration.

The method illustrated in FIG. 3 may be used to evaluate a porous structure, a temperature distribution, a pressure distribution and/or other characteristics related to a aqueous humor production or outflow. This structural evaluation may be carried out for initializing a working condition of the laser. This method may be further used to monitor and evaluate an effect of the modulated laser radiation on the eye. This laser effect evaluation may be carried out for controlling laser effect or to prevent damage induced by the laser on the eye.

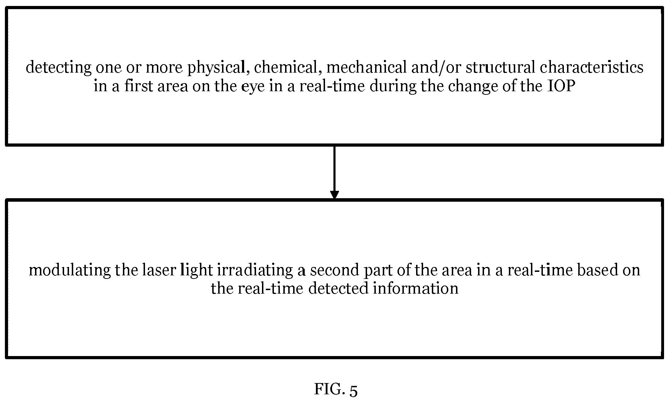

FIG. 5 is a flow chart illustrating a method for changing an IOP of an eye using a temporal and/or spatial modulated laser light comprising a treatment step, wherein the treatment step comprises:
a) detecting one or more physical, chemical, mechanical and/or structural characteristics in a first area on the eye in a real-time during the change of the IOP; and
b) modulating the laser light irradiating a second part of the area in a real-time based on the real-time detected information.

### Application scenario

In the following an example of the method for changing an IOP of an eye using a temporal and/or spatial modulated laser light is illustrated. This method corresponds to the method illustrated in FIG. 5 with extra optional steps.

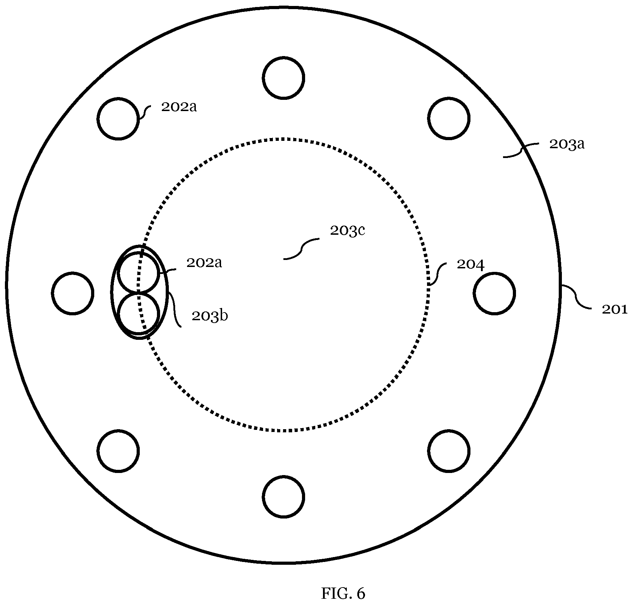

FIG. 6 is a schematic illustration of an eye 201 under a laser treatment or diagnosis according to an embodiment. This figure corresponds to a frontal view of the eye 201, where the cornea 203c is located in the middle of the figure and the sclera 203a is located outside the cornea 203c. The dotted line indicates the cornea limbus rim 204 which is located at the border between the cornea 203c and the sclera 203a. The trabecular meshwork 203b is located on the cornea limbus rim.

The laser system generates temporal and/or spatial modulated light and irradiate a first area 202a on the eye. As is illustrated in FIG. 6, the first area 202a may comprise several parts distributed on the eye. According to FIG. 6, eight parts of the first area 202a are located on the sclera, and two parts are on the TM. Other numbers of the parts may also be used depending on the concrete treatment cases. The parts may be separated from, connected with or overlap with each other.

The irradiations of different parts may be realized through different incident angles. For example, an irradiation on a ciliary body may be realized through a small incident angle. In this example, the incident laser light ray may penetrate through the sclera, and the light may be modulated considering both the sclera and the ciliary body. In another example, the irradiation of the ciliary body may be realized through a large incident angel. For example, the incident laser light ray may come from the side through the cornea. In this case the laser effect on the sclera may no longer considered.

In the example shown in FIG. 6 the eight parts of the first area on the sclera are arranged along the limbus rim. In the present disclosure, an arrangement of parts along the limbus rim may be understood as a circumferential arrangement along the limbus rim. The parts may have a certain distance from the limbus rim. They may be located outside or inside the limbus rim. They may be arranged on a circle which is confocal with the limbus rim, an ellipse surrounding the limbus rim and/or in another pattern.

The arrangement of the parts of the first area may be realized through the optical converting element 104. In another example, the optical converting element 104 may further provide a ring irradiation with a controlled diameter and width of the rim, and/or a spatial distribution of radiation modulated along the rim.

In this example, the improved approach for treatment of glaucoma is based on enhancing both conventional uveoscleral outflow by creating permeable pathways for water transport both through sclera and/or Schlemm's canal/ trabecular meshwork as a result of porous structure formation using laser radiation.

### Laser normalization of IOP in Eyes of Patients with high IOP.

The present disclosure provides a laser system suitable for forming a stabilized porous structure on the sclera and/or the superficial layer Schlemm's canal/ trabecular meshwork. In an example, porous structure may be formed on the sclera and/or Schlemm's canal/ trabecular meshwork without being stabilized. Forming porous structure on the sclera instead of /in addition to the Schlemm's canal/TM increase the utility of the uveoscleral pathway effectively and lowers IOP, in particular when the treatment on Schlemm's canal/TM alone no longer suffice.

However, if the pores are closed (healed), it can make the effect of lowering IOP short-lasting. Therefore, in an embodiment of the present disclosure, the laser system is further suitable to stabilize the porous structure, for example, through stabilizing the ions covering the bubble surface in the porous structures.

It is demonstrated that the small, submicron sized, stable gas bubbles arise in the sclera in response to laser irradiation. The stabilization of gas bubbles is due to repulsion of ions covering the bubble surface. That can stabilize the pores and prevent their collapse.

In an embodiment of the present disclosure, the laser system may further be suitable for destabilizing the porous structure, for example, through an overheating.

An overheating over 70 °C can anneal the stabilizing ions from the bubble surface hence triggering the collapse of the bubbles and pores in the tissue. This phenomenon may be used for the normalization of IOP for eyes with excessively low IOP.

Thus, to ensure the long-term clinical effect of the laser healing of glaucoma, and other technologies involving laser tissue modification, a more precise control over the laser heating regime may be beneficial.

In another embodiment a simultaneous (or sequential) laser irradiation is made on the ciliary body, on the trabecular region of the Schlemm's canal and/or on the sclera.

In an example, the proportion of coagulated ciliary body is determined using OCT in the process of exposure to the ciliary body. The change in the fluid flow, for example a flow of aqueous humor at each moment is calculated for an experimental change in the intensity of fluid release, for example an aqueous humor production, due to partial coagulation of the ciliary body.

The dimensions of the Schlemm's canal and pores in the trabecular region are controlled by OCT before and/or during laser irradiation.

In an example, the calculation of the desired size distribution of pores is carried out by a computer, based the measured volume of the ciliary body and the calculated change in the release of fluid by the ciliary body.

As a result, the desired porosity during exposure is calculated.

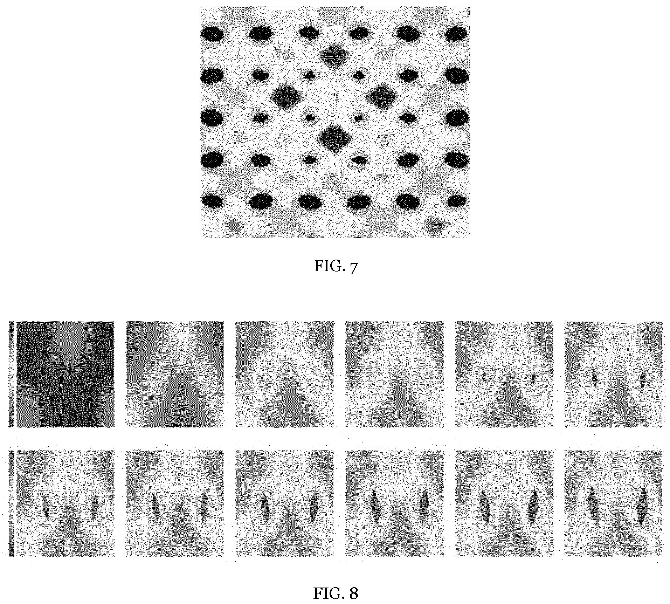

FIG. 7 is a graphical representation of the calculated size distribution of pores in trabecular meshwork as a result of laser irradiation of a rabbit eye. The pores with average size of 12 µm arise in the 160 µm area of the irradiated trabecular meshwork. That results in decreasing IOP by 2 mmHg.

FIG. 8 is a graphical representation of a calculated kinetic of the porous structure formation in a laser treated sclera of a human eye using a laser. The laser has a wavelength of 1560 nm, a laser spot diameter of 600 µm, a pulse duration of 200 ms, an average power of 0.8 W, a pulse repetition rate of 2.5 Hz, an exposure time of 42 s. The calculated maximal temperature is 59 °C, the average pore size is 9 µm and the desired diminishing in IOP is 6 mmHg.

The monitoring of the increase in porosity during irradiation is carried out by the measurement of backscattering light and/or using OCT during the changing of thickness of the sclera.

When the desired value and the monitored value of porosity coincide, the IOP may be then measured by a pneumatic device. In an exemplary IOP measurement by the pneumatic device, a recess on the cornea is created by a pneumatic pulse action. The recess size is measured using an OCT. If the IOP reaches a predetermined value, the irradiation stops.

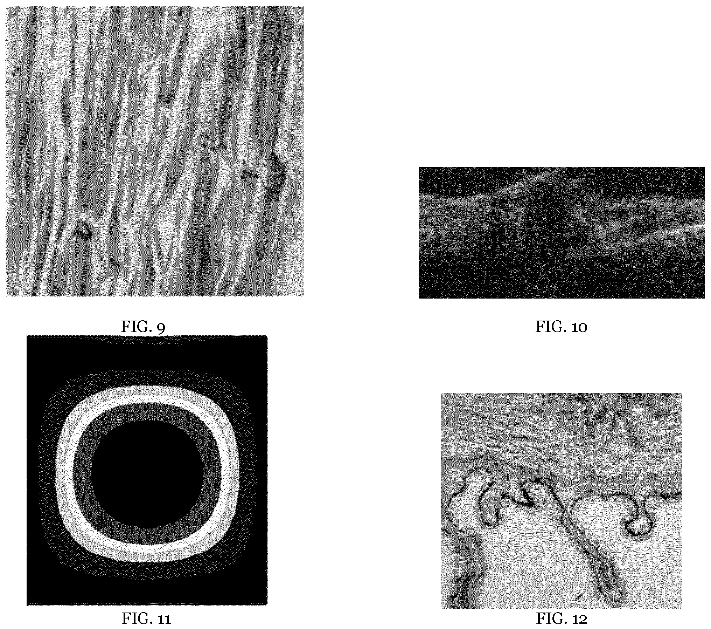

FIG. 9 is a histological image of laser-induced pores in sclera of a rabbit eye. The average width of the pores is 10 µm which allows to increase water transport trough the sclera up to 80 percent. The spatial size distribution and the formation dynamics of pores arisen as a result of laser irradiation of the areas of sclera and/or trabecular meshwork are calculated by a computer before laser irradiation and then measured in real time by the measurement of backscattering light and/or using OCT or an ultrasound device.

FIG. 10 is an ultrasound image of an irradiated sclera of a rabbit eye. The formation of pores in the sclera is manifested in real time by the increasing of a sclera thickness in the laser-irradiation area. The sclera thickness has increased by 220 µm, which represents an increase in water permeability trough the irradiated area of the sclera by 80 percent. The IOP has decreased by 3.2 mmHg.

During the abovementioned exemplary laser treatment, the temperature of the sclera may be monitored (e.g., by IR radiometry or photoacoustic sensor) and the laser parameters (power and/or time of the laser exposure) may be controlled so that the temperature is within a certain range (for example, between 45 and 65 °C).

FIG. 11 is a graphical representation of the calculated spatial distribution of temperature in trabecular meshwork during laser irradiation of a rabbit eye. The maximum temperature in the irradiation zone is 68 and 55°C, respectively, in the center and at the border of the laser spot 400 µm in diameter.

When laser-induced formation of porous structure in sclera and/or Schlemm's canal/ trabecular meshwork does not provide an adequate decrease of the IOP, the local laser coagulation of the ciliary body may be carried out. The laser induced coagulation of the ciliary body may be carried out in a real-time during the porous structure formation. However, due to the destructive nature of ciliary coagulation, the laser system may be configured to minimize the coagulation of the ciliary body.

FIG. 12 is a histological image of laser-induced coagulation of ciliary body of a rabbit eye. The desired coagulated volume was calculated as 3.3 mm³. A 1320nm transscleral laser has been regulated to achieve the desired coagulated volume. The size of the final coagulated zone is 1.5 mm which represents a final coagulated volume of 3.4 mm³. That allows to decrease the IOP by 3 mmHg.

### Laser normalization of IOP in Eyes of Patients with low IOP

There are two approaches to achieve this goal.
1) Closure of part of the stabilized pores in the sclera and trabecular region of the Schlemm's canal by increasing the temperature above 70 °C.

In an example, the calculation of the desired distribution of pore size is carried out, based on the change in the permeability of fluid such as aqueous humor through the sclera and trabecular meshwork.

The monitoring of the porosity decrease during irradiation is carried out by measuring the backscattering light and/or using OCT during the change of thickness of the sclera and the dimensions of the trabecular meshwork.

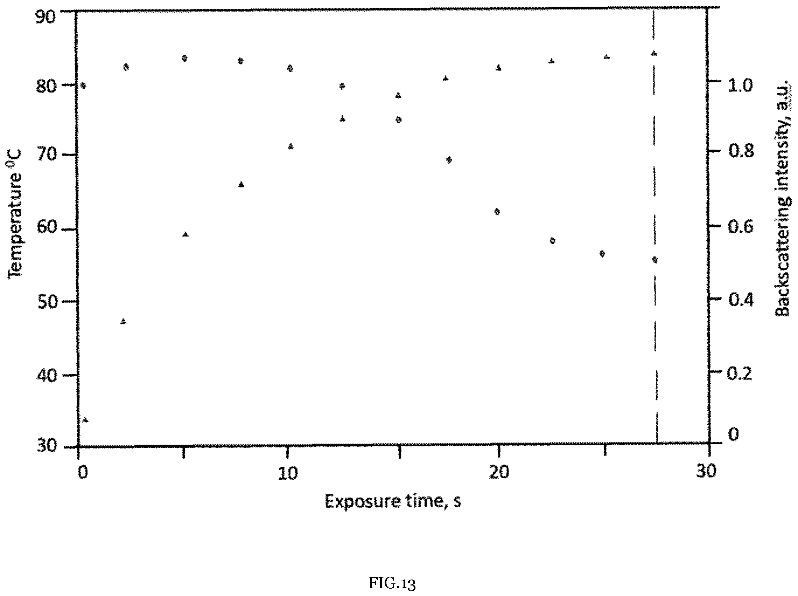

FIG. 13 is a graphical representation of the measured dynamic of temperature and backscattered light during laser irradiation of a sclera of a human eye. The x axis represents the time of irradiation. The triangular data points represent the temperature and the circular data points represent the intensity of backscattered light. The laser has a wavelength of 1560 nm, a power of 1.8 W, a spot diameter of 1 mm, a pulse duration of 200 ms, a pulse repetition rate of 2.5 Hz. At an exposure time of 27.5 s, indicated by the dashed line, a temperature of 83 °C and a twofold decrease in backscattering light intensity compared to the initial value have been observed. Such diminishing of backscattering light intensity is due to partial closure of the pores in the irradiated area of the sclera providing aqueous humor transport trough the sclera. The pores were stabilized with positive ions coating the surfaces of the pores. The pores are closed due to heating above 70 °C, which leads to the release of ions from the surface of the pores a local coagulation of the tissue near the pore surfaces. The value of exposure time is established by the feedback system based on calculation of aqueous humor transport in porous structure and the real time-data on temperature and IOP dynamics.

When the desired value and the monitored value of the porosity coincide, the IOP may be then measured by a pneumatic device. In an exemplary IOP measurement by the pneumatic device, a recess on the cornea is created by a pneumatic pulse action. The recess size is measured using the OCT. If the IOP reaches a predetermined value, the irradiation stops.

During the abovementioned exemplary laser treatment, the temperature of the sclera may be monitored (e.g., by IR radiometry or photoacoustic sensor) and the laser parameters (power and/or time of the laser exposure) may be controlled so that the temperature is within a certain range (for example, between 70 and 90 °C).

2) Laser activation and partial regeneration of the excessively damaged ciliary body.

In an example, the laser system may be suitable for generating a temperature and/or pressure condition to activate and/or regenerate damaged ciliary body. For a better healing effect, the activation/regeneration may be followed by an IOP measurement and (if necessary,) additional laser treatment after two or three months.

### Exemplary algorithm

An exemplary algorithm can be characterized through the mathematical problems and sub-problems that need to be solved by a large scale remote high-performance computer connected with a feedback controller for a real-time regulation of the laser system. The mathematical problems, sub-problems and tasks may include:
1. A 3D non-stationary heat problem for the spatial and temporal modulation of the laser heat source.
2. A 3D thermomechanical problem.
3. The 3D kinetics of laser induced bond breaking.
4. Kinetics of the porous structure formation, including a branching and merging of pores. FIG. 8 demonstrates an example of the pore size kinetics calculated in real time.
5. Kinetics of the tissue denaturation.
6. Kinetics of aqueous transport in a system of porous structure.

The calculation of the ratio between the effects of changing IOP due to laser exposure to the sclera, trabecular region and ciliary body is based on preoperative diagnostic data on IOP, the dimensions of pores in the sclera and trabecular meshwork, the size and condition of the ciliary body.

An algorithm used in the present disclosure should solve the inverse problem of determining the dosimetry of laser source to achieve a positive effect, such as desired IOP. The algorithm is based on the diagnostic data obtained before and during laser treatment of the eye with variation of the following parameters: laser wavelength, laser power, pulse duration, pulse repetition rate, laser spot diameter, distance between laser spots, quantity of pulses in a series, time interval between series, total exposure time.

Since people with thick and stiff corneas tend to have higher measured IOP than their actual IOP, the calculation algorithm may include adjustments based on measurements of corneal thickness and its Young's modulus.

The efficiency and safety of laser exposure is ensured by the choice and real time control of laser dosimetry, which provides certain temperature ranges, a certain size distribution of pores in the irradiation zone and a certain range of the thermomechanical stress amplitude during irradiation of the ciliary body.

Laser dosimetry for irradiation of sclera and trabecular meshwork is calculated to provide heating of the pores in sclera and/or trabecular meshwork to a temperature in the range of 65-90 °C for several seconds resulting in closure of the pores and diminishing of water transport in the irradiated area. Laser dosimetry for irradiation of ciliary body is calculated to provide thermomechanical action with frequency in the range of 0.2-5.0 Hz and amplitude of the oscillation pressure in the range of 3-20 kPa leading to activation of the regeneration processes in the irradiated area of the ciliary body.

For patients with elevated IOP, the ratio between the desired effects of irradiation of the sclera, Schlemm's canal, trabecular meshwork and ciliary body may be calculated based on diagnostic information obtained from all these targets and from the cornea. Laser dosimetry for irradiation is calculated to provide formation of pores of desired dimensions. The desired dimensions may be in the range of 5-20 µm in width and 10-100 µm long in the irradiated areas of sclera and trabecular meshwork; and in the range of 30 -300 µm for the Schlemm's canal.

The calculated temperatures in the irradiated areas, may be in the region of 45-65 °C for the sclera and trabecular meshwork, and 60-90 °C for the Schlemm's canal.

### Examples of successful treatments

The present disclosure has been implemented in certain preliminary experiments disclosed below. Although the disclosure has been implemented in these examples, these examples may contain extra steps, which should be not seen as restrictive to the present disclosure.

### First example: IOP diminishing in rabbit eyes.

This example involves a porous structure formation in sclera and trabecular meshwork followed by a local coagulation of ciliary body.

The experiments were performed in two eyes of a male New Zealand rabbit (weight, 3.1 kg).

The pre-operation diagnostics were conducted to determine the IOP, Young's modulus, stress distribution in cornea, sclera, and trabecular meshwork. The corneal Young's modulus was determined to be 0.30 +/- 0.05 MPa.

An average pore size of 3 microns in sclera and trabecular meshwork was measured by OCT. An intraocular pressure in the rabbit was measured using a pneumatic tonometer calibrated for the rabbit eye. Measurements were made without local anesthesia in restrained animals. The IOP of the rabbit was found to be 21.6 mmHg for the left eye and 21.7 mmHg for the right eye.

Desired diminishing of IOP was calculated to be 5 mmHg. The desired relative contributions of changes in IOP due to laser exposure to the trabecular meshwork, sclera and ciliary body were calculated to be 25%; 38% and 37% respectively.

The irradiation was performed in three steps.

### Step 1. Laser irradiation of trabecular meshwork, and IOP measurement.

For this step, the irradiation was conducted with a Tm fiber laser. The laser has a wavelength of 1920 nm, a laser spot diameter of 400 µm, a pulse duration of 5 ms, an average power of 2.0 W, and a pulse repetition rate of 20 Hz.

OCT imaging demonstrates a through channel in the trabecular meshwork of 420 and 460 µm in diameter in the left and right eye respectively.

IOP was measured to be 19.6 (left eye) and 19.2 (right eye) mmHg, which diminished by 2.0 and 2.5 mmHg respectively.

### Step 2. Laser irradiation of sclera.

For this step, the irradiation was conducted with a laser having a wavelength of 1440 nm, a laser spot diameter 400 µm, a pulse duration of 200 ms, an average power of 0.7 W, a pulse repetition rate of 2 Hz, 10 pulses in a series, a period between series of 5 s, and an exposure time 50 s.

FIG. 11 and FIG 7 demonstrate the calculated temperature field and spatial distribution of pores in laser irradiated sclera.

The real temperature field was measured during laser irradiation using optoacoustic method. The maximal temperature in the center of laser spot is 62 °C, the maximal temperature on the boundary of laser spot was 50 °C.

After irradiation, IOP was measured to be 16.3 mmHg (left eye) and 16.5 (right eye) mmHg. The Ultrasound imaging demonstrated the increase in sclera thickness manifesting formation of pores in the irradiated sclera (FIG. 10).

### Step 3. Local coagulation of ciliary body,

For this step, the irradiation was conducted with a laser having a wavelength of 1320 nm, a laser spot diameter of 200 µm, a pulse duration of 1 ms, an average power of 1.4 W, a pulse repetition rate of 5 Hz, 20 pulses in a series, a period between series of 5 s, an exposure time of 40 s.

The calculated desired size of coagulated ciliary body was 65 µm. The estimated diminishing in IOP was 3 mmHg. Real diminishing after this step was 2.8 (left eye) and 3.3 mmHg (right eye) respectively.

The IOP was measured for rabbits in an hour and in a month after all three stages of treatments. In an hour, IOP was 13.5 (left eye) and 13.2 (right eye) mmHg, diminished by 8.1 and 8.5 mmHg respectively.

In a month after irradiation, the final IOP was 13.6 (left eye) and 13.6 (right eye) mmHg respectively. So, the final IOP diminishing was 8.0-8.1 mmHg (including a contribution of 24 % after the first step, a contribution of 39% after the second step and a contribution of 37% after the third step).

OCT imaging shows channels in the trabecular meshwork with a size of about 380 +/-30 µm in diameter, and pores with average size of 9 µm in sclera.

Then rabbits were sacrificed for histological analysis. The results demonstrate a formation of pores in the irradiated sclera with average pore size of 10 µm (FIG. 9), and formation of a 60 +/-10 µm coagulation area in the ciliary body (FIG. 12).

So, the first example demonstrates a predictable decrease in IOP in the eyes of living rabbits due to subsequent irradiation and the formation of pores in the trabecular meshwork and sclera and local coagulation of the ciliary body. In the eyes of live rabbits, the stability of the decrease in IOP was maintained for at least two months.

### Second example: Diminishing IOP in human eye with laser irradiation of sclera and ciliary body in both eyes.

Pre-operation diagnostics show an initial IOP of 32 mmHg (left eye) and 31 mmHg (right eye) respectively. An OCT diagnostic of sclera shows an average pore size of 4 µm. The thickness of the cornea was measured with OCT. Mechanical properties, including Young's modulus and a stress distribution in cornea, sclera and at or in the vicinity of trabecular meshwork were measured with OCE using the pneumatic device.

Irradiation was performed in two steps.

### Step 1. Irradiation of the sclera.

For this step, the irradiation was conducted with a laser having a wavelength of 1560 nm, a laser spot diameter of 600 µm, a pulse duration of 200 ms, an average power of 0.8 W, a pulse repetition rate of 2.5 Hz, 10 pulses in a series, a period between series of 5 s, an exposure time of 42 s. The optical delivery element 102 and an optical converting element 104 enabled a formation of 8 parts of irradiated area with a diameter of 600 µm each at a distance of 1 mm from the limbus of the eye. (FIG. 6).

The following characteristics were calculated: a maximal temperature of 59 °C; an average pore size increasing with time (FIG. 8) and reaches 9 µm at 45 s of irradiation; a desired diminishing in IOP by 6.2 mmHg.

Temperature measurement was performed during laser irradiation using an IR optical radiometer. The maximal temperature was 59 +/-0.5 °C.

After an irradiation of sclera, the IOPs were measured to be 26.2 mmHg (left) and 26.0 (right), and the average pore size were measured to be 8 µm (left) and 9 µm (right eye).

### Step 2. Irradiation of ciliary body.

For this step, the irradiation was conducted with a laser having a wavelength of 1320 nm, a laser spot diameter of 200 µm, a pulse duration of 1 ms, an average power of 1.5 W, a pulse repetition rate of 5 Hz, 20 pulses in a series, a period between series of 5 s, an exposure time of 45 s.

The following characteristics were calculated: a zone of coagulated ciliary body of 90 µm; an estimated diminishing in IOP of 10 mmHg; a maximal temperature of 82 °C. Temperature measurement during irradiation showed 82 +/-1 °C.

The IOPs were controlled several times after the treatment: for the left eye, IOP was 15.5 mmHg directly after the treatment, 16.5 mmHg in 6 months, and 16.8 mmHg in 12 months; for the right eye IOP was 15.2 mmHg directly after the treatment, 16.1 mmHg in 6 months, and 16.4 mmHg in 12 months.

This example demonstrates a long-term predictable decrease in IOP in the human due to subsequent irradiation and the formation of pores in sclera and local coagulation of the ciliary body. In both human eyes, the stability of normal IOP was maintained for at least twelve months.

### Third example: Normalization of IOP in human eye with low IOP.

The treatment was conducted on a right human eye after a glaucoma surgery, with chronic eye inflammation.

The initial IOP of the right eye was measured to be 8 mmHg.

Diagnostics (OCT) show large pores (average pore size of 27 microns) in sclera and a pronounced inflammation in ciliary body.

OCE measurements show reduced Young's modulus values, for example 0.1 MPa and 0.3 MPa for the cornea and sclera, respectively.

According to an IOP measurement, the left eye had an IOP of 15 mmHg with no visible inflammation. The average pore size in the left eye is about 10 microns.

Temperature measurement was performed using an IR radiometer.

Light scattering measurement was performed using an optical detector.

Thermomechanical oscillations measurements were performed with an optoacoustic detector.

### Irradiation of the right eye.

The dosimetry of laser source to achieve a positive effect, such as desired IOP was calculated. The algorithm is based on the diagnostic data obtained before and during laser irradiation of the eye with variation of the following parameters: laser wavelength, power, pulse duration, pulse repetition rate, laser spot diameter, quantity of pulses in a series, time interval between series, total exposure time.

Laser irradiation was performed in two steps.

### Step. 1. Closure of pores in sclera.

For this step, the irradiation was conducted with a laser having a wavelength of 1560 nm, a power of 1.8 W, a laser spot diameter of 1 mm, a pulse duration of 200 ms, a pulse repetition rate of 2.5 Hz, an exposure time of 32 s. The laser parameters were established by the feedback controller based on measurement of backscattering light during irradiation of the sclera (FIG. 13).

An optical delivery element 102 and an optical converting element enabled a formation of 6 parts of irradiated area with a diameter of 1 mm each at 2 mm from the limbus of the eye. (FIG. 6).

Temperature measurement shows a heating up of the sclera to 82 +/-1 °C.

After laser irradiation of sclera, the IOP was measured to be 11.5 mmHg, and the average pore size of 9 µm was measured with an OCT.

The Young's modulus of the sclera measured with OCE was 0.45 MPa (which means it returned to the normal range).

### Step 2. Irradiation of ciliary body with modulated laser light to activate regeneration processes and diminish inflammation.

For this step, the irradiation was conducted with a laser having a wavelength of 1320 nm, a pulse duration of 200 ms, a laser spot diameter of 1.0 mm, a laser power of 0.4 W, a pulse repetition rate of 1 Hz, 4 series of laser pulses with 10 pulses in a series, a period between series of 10 s, exposure time of 70 s.

Temperature measurement during irradiation shows a maximal temperature of 48 °C. Measurements of the oscillation pressure show the pressure in the range of 8 -12 kPa. These lead to the activation of the regeneration processes in the irradiated area of the ciliary body.

The IOP of the right eye was measured to be 11.7 mmHg directly after laser treatment, 15.2 mmHg in one year after treatment, 15.4 mmHg in two years after treatment, and 15.5 mmHg in three years after treatment.

According to an examination using OCT, the average pore size was 9 µm. No inflammations, no large pores were observed after the laser treatment.

The IOP of left eye was measured to be 15 mmHg directly after laser treatment, 15.4 mmHg in one year after treatment, 15.6 mmHg in two years after treatment, and 15.7 mmHg in three years after treatment.

This example demonstrates a recovery in normal IOP in the human eye due to diminishing average pore size (closure of large pores) in the sclera, cessation of inflammation and activation of regeneration processes in the ciliary body. The long-term stability of normal IOP was maintained for at least three years after treatment.

Embodiments of methods and laser system according to the present disclosure may show the following advantages:
1. Several (three) targets may be treated concurrently: ciliary body, sclera and Schlemm's canal with trabecular meshwork. Clear mechanisms of laser action are the advantage of the method. The goal is the control the IOP for patients with unnormal (elevated or low) IOP by the laser effect on (i) the volume and productivity of the ciliary body, (ii) the permeability of the sclera, and (iii) permeability of Schlemm's canal and trabecular meshwork.
2. Reversibility of lowering IOP due to closure of pores in the sclera and (or) trabecular meshwork due to a temporary increase in temperature over 70 °C.
3. Feedback control system based on measurement of several parameters (temperature, electrical impedance, water permeability, micropore formation in sclera and/or trabecular meshwork, mechanical properties of cornea and sclera, light backscattering, and the dimensions of the coagulation zone in the ciliary body). Information on mechanical properties of cornea is used for estimation of final desired characteristics (size distribution) of micropores because lower mechanical properties of the cornea can increase the amount of intraocular fluid and potentially lead to the development of glaucoma. In addition, a small number of large pores and many small pores with the same total cross-sectional area give different IOP values and different IOP stability. Therefore, the control of pore size distribution and temperature range are of great importance to provide long-term normalization of IOP.
4. An automatic device with real time control of laser dosimetry is controlled with remote high-performance computer.
5. The method and device can be used both for elevated and/or low IOP.

The description of the specific embodiments and the Figures merely serves to illustrate the techniques of the present disclosure and the advantageous effects associated therewith but should not imply any limitation. The scope of the disclosure is to be inferred from the appended claims.

### List of reference signs

- 101: laser source
- 102: optical delivery element
- 103: working tool
- 104: optical converting element
- 105: detecting element
- 106: feedback controller
- 106a: electronic unit
- 106b: feedback control element
- 106c: remote hybrid computational facility, and/or the remote quantum computer
- 201: eye
- 202a: first area
- 202b: second area
- 203a: sclera
- 203b: trabecular meshwork
- 203c: cornea
- 203d: Schlemm's canal
- 203e: ciliary body
- 203f: iris
- 203g: lens
- 204: cornea limbus rim
- 205a: conventional pathway
- 205b: uveoscleral pathway

## Claims

1. A laser system suitable for changing an IOP of an eye (201), the laser system comprising:
a laser source (101);
a feedback controller (106), configured to regulate a dosimetry of the laser source (101) to produce spatially and/or temporally modulated laser light;
a first optical delivery element (102), configured to guide the spatially and/or temporally modulated laser light to irradiate a first area (202a) on the eye (201); and a detecting element (105), configured to detect one or more physical, chemical, mechanical and/or structural characteristics in a second area (202b) on the eye (201) in a real-time during the change of the IOP,
wherein the feedback controller (106) is configured to regulate the dosimetry of the laser source (101) in a real-time based on the real-time detected information pertaining to the one or more physical, chemical, mechanical and/or structural characteristics in the second area (202b).

2. The laser system of claim 1,
wherein the first area (202a) comprises a part on a sclera (203a) of the eye (201), and wherein the laser source (101) is regulated to modify a porous structure on the sclera (203a).

3. The laser system of claim 2,
wherein the modulated laser light is suitable for achieving and/or maintaining a first temperature range and/or a second temperature range in the first area (202a), wherein the porous structure is stabilized in the first temperature range, and the porous structure is destabilized in the second temperature range.

4. The laser system of claims 2 or 3,
wherein the feedback controller (106) is configured to calculate a flow rate of fluid through the porous structure on the sclera (203a) based on the detected information.

5. The laser system of any one of the preceding claims,
wherein the second area (202b) comprises a part on the cornea (203c), wherein the detected information pertains to one or more mechanical characteristics of the cornea (203c),
and wherein the feedback controller (106) is configured to acquire an overall mechanical property of the eye (201), comprising a mechanical property of the trabecular meshwork (203b), a mechanical property of a superficial layer of the Schlemm's canal (203d), a mechanical property of a ciliary body (203e) and/or a mechanical property of the sclera (203a).

6. The laser system of claim 5.
wherein the detecting element (105) comprises an OCE device and a pneumatic device combined with the OCE device, wherein the detecting element (105) continuously measures the one or more mechanical characteristics of the cornea (203c).

7. The laser system of any one of the preceding claims,
wherein the first area (202a) comprises a part on a ciliary body (203e) of the eye (201),
and wherein the laser source (101) is regulated to activate one or more cells on the ciliary body (203e).

8. The laser system of claim 7,
wherein the modulated laser light is suitable for generating a temperature and/or pressure condition to activate the one or more cells.

9. The laser system of claim 7 or 8,
wherein the modulated laser light is suitable for generating a thermomechanical wave, which can propagate to a third area outside the first area (202a), and
wherein the one or more cells are in the third area.

10. The laser system of any one of the preceding claims,
wherein the first area (202a) comprises at least two of the following:
a) a part on a sclera (203a) of the eye (201);
b) a part at and/or in the vicinity of a Schlemm channel and/or a trabecular meshwork (203b) of the eye (201); and
c) a part on the ciliary body (203e) of the eye (201),
and wherein the feedback controller (106) is configured to calculate a desired relative contribution of the two parts on normalizing the IOP, and
wherein the dosimetry of the laser source (101) is regulated to achieve the desired relative contribution.

11. The laser system of claim 10,
wherein the feedback controller (106) is configured to determine, based on the detected information, whether one of the parts has been treated before,
and wherein when the feedback controller (106) determines that the one of the parts has been treated before, the feedback controller (106) is configured to correct the desired relative contribution by reducing the desired contribution of the treated part.

12. The laser system of any one of the preceding claims, wherein
the feedback controller (106) comprises and/or is coupled to a high-performance computer, a hybrid quantum-classical computational facility, and/or a quantum computer (106c); and/or
the feedback controller (106) comprises and/or is connected to a storage device, the storage device storing an offline settings table, wherein the settings table is calculated by a high-performance computer, a hybrid quantum-classical computational facility, and/or a quantum computer (106c).

13. A method, comprising:
a) detecting one or more physical, chemical, mechanical and/or structural characteristics in a first area (202b) on an eye (201);
b) processing the detected information pertaining to the physical, chemical, mechanical and/or structural characteristics in the first area (202b) on the eye (201); and
c) acquiring a property of a porous structure change on the eye (201) and/or an activation of a ciliary body (203e) regeneration of the eye (201), in a real-time during the porous structure change and/or the activation of the ciliary body (203e) regeneration.

14. The method of claim 13,
wherein the first area (202b) comprises a part on a cornea (203c) of the eye (201), wherein the detected information pertains to the mechanical characteristics of the cornea (203c),
wherein the acquiring the property of a porous structure change on the eye (201) and/or an activation of a ciliary body (203e) regeneration of the eye (201), in a real-time during the porous structure change and/or the activation of the ciliary body (203e) regeneration comprises:
acquiring an overall mechanical property of the eye (201), comprising a mechanical property of the trabecular meshwork (203b), a mechanical property of a superficial layer of the Schlemm's canal (203d), a mechanical property of a ciliary body (203e) and/or a mechanical property of the sclera (203a).

15. The method of claim 13 or 14, wherein the processing of the detected information is performed in real time using a high-performance computer, a hybrid quantum-classical computational facility, and/or a quantum computer (106c).
